# EUROPEAN PATENT SPECIFICATION

(11) **EP 1 478 364 B1**
(45) Date of publication and mention of the grant of the patent: **16.09.2009**
(21) Application number: 02802626.8
(22) Date of filing: 21.10.2002
(51) Int. Cl.: A61K 31/505, A61K 31/44, A61K 31/38, A61K 31/415, A61K 31/42, A61K 31/435, A61P 31/04, C07D 409/12, C07D 231/40, C07D 333/38, C07D 307/68

(54) **SELECTIVE ANTIBACTERIAL AGENTS**
SELEKTIVE ANTIBAKTERIELLE MITTEL
AGENTS ANTIBACTERIENS SELECTIFS

(30) Priority: 07.11.2001 WO PCT/EP01/12875; 08.03.2002 US 94301
(43) Date of publication of application: 24.11.2004
(73) Proprietor: Quonova, LLC, Melbourne, FL 32901 (US)
(72) Inventor: KRAMER, Bernd, 52080 Aachen (DE); AMMENDOLA, Aldo, 81375 München (DE); SAEB, Wael, 82152 Planegg-Martinsried (DE)
(74) Representative: Vossius & Partner
(86) International application number: PCT/EP2002/011760
(87) International publication number: WO 2003/039549

(56) References cited:
- WO-A-01/85664
- WO-A-98/57618
- WO-A-99/27786
- SCHAEFER A L ET AL: "QUORUM SENSING IN VIBRIO FISCHERI: PROBING AUTOINDUCER-LUXR INTERACTIONS WITH AUTOINDUCER ANALOGS" JOURNAL OF BACTERIOLOGY, WASHINGTON, DC, US, vol. 178, no. 10, May 1996 (1996-05), pages 2897-2901, XP000884120 ISSN: 0021-9193
- LEADBETTER JARED R ET AL: "Metabolism of acyl-homoserine lactone quorum-sensing signals by Variovorax paradoxus" JOURNAL OF BACTERIOLOGY, WASHINGTON, DC, US, vol. 182, no. 24, December 2000 (2000-12), pages 6921-6926, XP002208346 ISSN: 0021-9193

## Description

The present invention relates to the use of compounds such as amide, carbazide, hydrazide, urea, and guanidine derivatives as selective inhibitors of bacterial pathogens. In particular the invention refers to a family of compounds that block the quorum sensing system of Gram-negative bacteria, a process for their manufacture, pharmaceutical compositions containing them and to their use for the treatment and prevention of microbial damages and diseases, in particular for diseases where there is an advantage in inhibiting quorum sensing regulated phenotypes of pathogens.

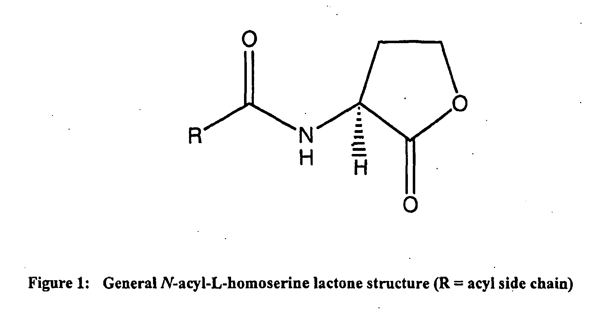

Many microorganisms, including bacteria, fungi, protozoa and algae cause severe damages or diseases in different areas such as industry, agriculture, environment and medicine. Especially bacteria as human pathogens cause tremendous costs in public health systems worldwide. The continuing emergence of multiple-drug-resistant bacterial strains has necessitated finding new compounds that can be used in antibacterial treatment. There are two broad strategies for the control of bacterial infection: either to kill the organism or to attenuate its virulence such that it fails to adapt to the host environment. The latter approach has, however, lacked specific targets for rational drug design. The discovery that Gram-negative bacteria employ a signal transduction pathway comprising a small molecule to globally regulate the production of virulence determinants offers such a novel target.
A wide variety of Gram-negative bacteria produce *N*-acyl-L-homoserine lactone (AHL or HSL, Figure 1) derivatives as signal molecules in intercellular communication. These molecules, also referred to as "pheromones" or "quoromones", comprise a homoserine lactone moiety linked to an acyl side chain. Bacteria use this signaling system to monitor their population cell density in a process referred to as "quorum sensing". In each cell of a population an HSL synthase from usually the Luxl family of proteins produce a low basal level of diffusible HSLs. The HSL concentration increases with bacterial population density until a threshold concentration is reached which results in expression of various HSL-dependent genes through an HSL-receptor protein belonging generally to the LuxR family of transcriptional regulators. This HSL-receptor protein complex serves not only as positive transcription regulator of quorum sensing regulated genes but also as positive regulator for the HSL synthesis itself. Therefore, the entire system is amplified via a process of autoinduction.

This system was first discovered in the bioluminescent marine bacteria *Vibrio harveyi* and *V. fischeri* where it is used to control bioluminescence expression. In recent years it has become apparent that many Gram-negative bacteria employ one or more quorum sensing systems comprising HSL derivatives with different acyl side chains to regulate in a cell-density dependent manner a wide variety of physiological processes such as swarming motility, biofilm formation, pathogenicity, conjugation, bioluminescence or production of pigments and antibiotics (Table 1, for reviews and further references see, e.g.: Fuqua et al., Ann. Rev. Microbiol. 50:727-51, 1996; Fuqua & Greenberg, Curr. Opinion Microbiol. 1:183-89, 1998; Eberl, Syst. Appl. Microbiol. 22:493-506, 1999; De Kievit & Iglewski, Infect. Immun. 68:4839-49,2000).

**Table 1: Summary of HSL-based quorum sensing systems**

| **Bacterium** | **Regulatory proteins** | **Major HSL** | **HSL-regulated phenotype** |
|---|---|---|---|
| *Aeromonas hydrophila* | AhyR, AhyI | C4-HSL | Extracellular protease, biofilm formation |
| *Aeromonas salmonicida* | AsaR, AsaI | C4-HSL | Extracellular protease |
| *Agrobacterium tumefaciens* | TraR, TraI | 3-oxo-C8-HSL | Conjugal transfer |
| *Burkholderia cepacia* | CepR, CepI | C8-HSL | Protease, lipase, ornibactin synthesis, biofilm formation, swarming motility |
| *Chromobacterium violaceum* | CviR, CviI | C6-HSL | Antibiotics, violacein, exoenzymes, cyanide |
| *Enterobacter agglomerans* | EagR, EagI | 3-oxo-C6-HSL | Unknown |
| *Erwinia carotovora* | CarR, (CarI) ExpR, Expl | 3-oxo-C6-HSL | Carbapenem antibiotics, oxoenzyme production |
| *Erwinia chrysanthemi* | ExpR, ExpI (EchR, EchI) | 3-oxo-C6-HSL | Pectinase expression |
| *Escherichia coli* | SdiA | Unknown | Cell division, virulence factor production |
| *Nitrosomonas europaea* | Unknown | 3-oxo-C6-HSL | Emergence from lag phase |
| *Obesumbacterium proteus* | OprR, OprI | 3-oxo-C6-HSL | Unknown |
| *Pantoea stewartii* | EsaR, EsaI | 3-oxo-C6-HSL | Exopolysaccharide production, virulence factor production |
| *Pseudomonas aeruginosa* | LasR, LasI | 3-oxa-C12-HSL | Extracellular virulence factors, Xcp, biofilm formation, RpoS, RhIR |
| *Pseudomonas aeruginosa* | RhIR, RhlI | C4-HSL | Extracellular virulence factors, cyanide, lectins, pyocyanin, rhamnolipid, type 4 pili, twitching motility |
| *Pseudomonas aureofaciens* | PhzR, PhzI | C6-HSL | Phenazine antibiotics |
| *Pseudomonas fluorescens* | HdtS | 3-hydroxy-7-*cis-C 14-HSL* | Unknown |
| *Ralstonia solanacearum* | SolR, SolI | C8-HSL | Unknown |
| *Rhizobium etli* | RaiR, RaiI | 7 HSLs | Root nodulation |
| *Rhizobium leguminosarum* | RhiR | 3-hydroxy-7-*cis*-C14-HSL | Nodulation, bacteriocin, stationary phase survival |
| *Rhizobium leguminosarum* | RhiR, RhiI | C6-HSL, C8-HSL | rhizome interactions |
| *Rhodobacter sphaeroides* | CerR, CerI | 7-cis-C14-HSL | Clumping factor |
| *Serratia liquefaciens* | SwrR, SwrI | C4-HSL | Swarming motility, protease, serrawettin W2, lipase |
| *Vibrio anguillarum* | VanR, VanI | 3-oxo-C10-HSL | Unknown |
| *Vibrio anguillarum* | VanM, VanN | C6-HSL, 3-hydroxy-C6-HSL | Unknown |
| *Vibrio fischeri* | LuxR, LuxI | 3-oxo-C6-HSL | Bioluminescence |
| *Vibrio harveyi* | LuxM, LuxN | 3-hydroxy-C4-HSL | Bioluminescence, PHB synthesis |
| *Xenorhabdus nematophilus* | Unknown | 3-hydroxy-C4-HSL | Virulence |
| *Yersinia enterocolitica* | YenR, YenI | C6-HSL, 3-oxo-C6-HSL | Unknown |
| *Yersinia pestis* | YpeR, YpeI | Unknown | Unknown |
| *Yersinia pseudotuberculosis* | YpsR, YpsI | 3-oxo-C6-HSL | Motility, clumping |
| *Yersinia pseudotuberculosis* | YtbR, YtbI | C8-HSL | Unknown |
| *Yersinia ruckeri* | YukR, YukI | Unknown | Unknown |

With regard to bacteria that utilize HSL-based quorum sensing as part of their lifestyle, *Pseudomonas aeruginosa* is perhaps the best understood in terms of the role quorum sensing plays in pathogenicity. In this human opportunistic pathogen, which causes nosocomial infections in immunocompromized patients and has an extremely high potential to develop resistance mechanisms against traditional antibiotic treatment, production of many virulence factors including several proteases, exotoxin A, rhamnolipid, pyocyanin, cyanide and chitinasc is regulated by two interlinked quorum sensing circuits. Moreover, it has been demonstrated that this signaling system is involved in the ability of *P. aeruginosa* to form biofilms (Davies et al., Science 280:295-8, 1998). Recently Huber et al. (Microbiology 147:2517-28, 2001) demonstrated that biofilm formation and swarming motility of *Burkholderia cepacia,* like *P*. *aeruginosa* a human opportunistic pathogen, is also dependent on an HSL-based quorum sensing system.
Biofilms are defined as an association of microorganisms growing attached to a surface and producing a slime layer of extracellular polymers in which the microbial consortia is embedded in a protective environment (for a review see: Costerton et al., Ann. Rev. Microbiol. 49:711-45, 1995). Biofilms represent a severe problem as bacteria integrated in such a polymer matrix develop resistance to conventional antimicrobial agents. *P. aeruginosa* cells, for example, growing in an alginate slime matrix have been demonstrated to be resistant to antibiotics (e.g., aminoglycosides, β-lactam antibiotics, fluoroquinolones) and disinfectants (Govan & Deretic, Microbiol. Rev. 60:539-74, 1996). Several mechanisms for biofilm-mediated resistance development have been proposed (Costerton et al., Science 284:1318-22, 1999).
In most natural, clinical and industrial settings bacteria are predominantly found in biofilms. Drinking water pipes, ship hulls, teeth or medical devices represent typical surfaces colonized by bacteria. On the one hand biofilms decrease the life time of materials through corrosive action in the industrial field, a process also referred to as "biofouling". Furthermore, microbial biofilms growing for example on ship hulls increase fuel consumption through enhanced frictional resistance and simultaneously reduce maneuverability. On the other hand two thirds of all bacterial infections in humans are associated with biofilms (Lewis, Antimicrob. Agents Chemother. 45:999-1007, 2001).
*Pseudomonas aeruginosa,* for example, forms infectious biofilms on surfaces as diverse as cystic fibrosis lung tissue, contact lenses, and catheter tubes (Stickler et al., Appl. Environm. Microbiol. 64:3486-90, 1998). *Burkholderia cepacia* also forms biofilms in lungs of cystic fibrosis patients and is a major industrial contaminant (Govan et al., J. Med. Microbiol. 45:395-407, 1996). Since biofilm formation of both organisms is demonstrated to require an HSL signaling system, inhibition of their quorum sensing systems would result in an impaired ability to form biofilms and therefore in an increased susceptability to antibacterial treatment.
The discovery that a wide spectrum of organisms use quorum sensing to control virulence factor production and other phenotypes such as biofilm formation makes it an attractive target for antimicrobial therapy. Pathogenic organisms using this signaling system to control virulence could potentially be rendered avirulent by blocking this cell-cell communication system. In contrast to traditional antibiotics, the risk of resistance development seems to be very low, since quorum sensing blocking agents would not kill the organism but disturb signal transduction pathways. There are several possibilities of interrupting the quorum sensing circuit.
For example, plants expressing an HSL-lactonaas enzyme originally derived from *Bacillus sp.* have been demonstrated to quench pathogen quorum sensing signaling and to significantly enhance resistance to *Erwinia carolovora* infections (Dong et al., Nature 411:813-7, 2001). An alternative way to block cell signaling could be to interrupt the HSL synthesis by using analogs of HSL precursors.
However, the most promising possibility to block quorum sensing is to take advantage of the unique specificity the HSLs and HSL-receptor proteins show for one another. The ability of homoserine lactone-based analogs to inhibit activation of HSL-receptor proteins has already been demonstrated in a number of bacteria including *Vibrio fischeri* (Schaefer et al., J. Bacteriol. 178:2897-901, 1996), *Agrobacterium tumefaciens* (Zhu et al., J. Bacteriol. 180:5398-405, 1998), *Chromobacterium violaceum* (McLean et al., Microbiology 143:3703-11, 1997), *Aeromonas salmonicida* (Swift et al., J. Bacteriol. 179:5271-81, 1997) and *Pseudomonas aeruginosa* (Pesci et al., J. Bacteriol. 179:3127-32, 1997). However, none of these compounds have been developed as antimicrobial agents, e.g. in medical therapy, so far.
The only described non-HSL-based antimicrobials which are supposed to interfere specifically with HSL-regulated processes are halogenated furanone derivatives which are structurally similar to HSLs and have been isolated from red marine algae *Delisea pulchra* (WO 96/29392). Additionally, these substances have been demonstrated to inhibit also Gram-positive bacteria (WO 99/53915). However, the use of most of these compounds is limited due to their toxicity making them unsuitable for veterinary and medical applications.
Many target genes involved in biofilm formation, methods of screening for compounds to control biofilm development and HSL-based compositions to prevent biofilm formation have been described (WO 99/55368, WO 98/57618, WO 99/27786, WO 98/58075), but until now no promising antibacterial drug candidate has been developed that is capable of inhibiting biofilm formation in different areas, preferentially in the medical field.
WO 99/27786 relates to the control of biofilm formation. In one aspect, it relates to the use of a group of compounds for treating and/or preventing bacterial infections in humans or animals by control of biofilm formation.
WO 01/85664 relates to a method for regulating the activity of an autoinducer-2 receptor comprising contacting an autoinducer-2 receptor with an Al-2 agonist or antagonist compound.
WO 98/57618 relates to a biofilm removing or inhibiting composition comprising an amount of a compound selected from the group consisting of blockers of N-(3-oxododecanoyl) L- homoserine lactone (OdDHL), and analogs of butyryl L- homoserine lactone (BHL), and mixtures thereof, and a vehicle or carrier, wherein the amount of the compound is effective to remove or disrupt a bacterial biofilm or inhibit normal biofilm formation.
Schaefer et al (Quorum sensing in Vibrio fischeri: probing autoinducer-LuxR interactions with autoinducer analogs in J Bacteriol., 1996, May; 178(10): 2897-2901) report that the *Vibrio fischeri* luminescence genes are activated by the transcription factor LuxR in combination with a diffusible signal compound, N-(3-oxohexanoyl) homoserine lactone, termed the autoinducer.
Leadbetter et al (Metabolism of Acyl-Homoserine Lactone Quorum-Sensing Signals by Variovorax paradoxus in Journal of Bacteriology, Dec. 2000, p. 6921-6926, Vol. 182, No. 24) report that Acyl-homoserine lactones (acyl-HSLs) serve as dedicated cell-to-cell signaling molecules in many species of the class Proteobacteria.

It is an object of the present invention to provide compounds blocking specifically quorum sensing regulated processes without inhibiting bacterial growth. Furthermore, these compounds should not be structural derivatives of the homoserine lactone family of regulatory compounds and should not exhibit any toxic properties.
Accordingly, we have been able to find compounds that can significantly reduce virulence gene expression and biofilms formation of several human pathogens. In contrast to the furanones the compounds of this invention do not show any toxic effect and are therefore suitable for applications in a wide area. Such applications could be the use of the compounds for instance as new antibiotic therapeutics, disinfectants, antifouling coatings or coatings of medical devices. In contrast to traditional antibacterial agents (like amide or 1,2-acylhydrazine derivatives in WO 01/51456; for the synthesis of amide or 1,2-acylhydrazine derivatives see also EP 638545 and EP 982292), the compounds of the present invention do not kill the microorganisms, but render them avirulent. The advantage of this alternative strategy is that the emergence of bacterial resistance against such antimicrobials is extremely improbable.

In general, the present invention provides compounds selectively modulating bacterial cell-cell communication. Through inhibition of this communication system the expression of many HSL-dependent virulence genes and other phenotypes like swarming motility and biofilm formation are significantly reduced or completely abolished rendering a bacterial population more susceptible to the host immune-response or to treatment with traditional antibacterial agents.
Thus, in one aspect, the invention refers to a method for inhibiting an HSL-regulated process in a microorganism by exposing the microorganism to a new class of compounds with an inhibitory effect on bacterial signaling.
The present invention therefore refers to compounds of the general Formula (I) 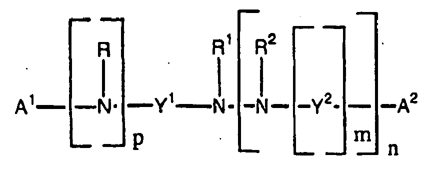 wherein
- R: is H;
- R¹: is H;
- R²: is H;
A¹ and A² each independently represent an C₁-C₂₀-alkyl group which may contain one or more group(s) Z, or a monocyclic or polycyclic aromatic or non-aromatic ring system which may contain one or more group(s) X, and in case of a polycyclic ring system, said system contains at least one aromatic ring;
- Z: is selected from the group consisting of S, O, N, NR⁴, CO, CO₂, CS; SO or SO₂
- X: is selected from the group consisting of S, O, N, NR⁴, SO or SO₂;
said substituted ring system carriers a substituent R³ on one or more of the carbon atoms of said ring system;
said substituted C₁-C₂₀-alkyl group carries a substituent R³ on one or more of the carbon atoms of said alkyl group;
- R³: is independently H, OR⁴, SR⁴, hydroxyalkyl, hydroxyalkylamino, cycloalkyl, halogen, haloalkyl, haloalkyloxy, NO₂, CN, SO₂NR⁴R⁵, CO₂NR⁴R⁵, COR⁴, CO₂R⁴, SO₂R⁴, SO₃R⁴, NR⁴R⁵, alkyl, aryl or heteroaryl;
- R^{3'}: is independently H, OR⁴, SR⁴, hydroxyalkyl, hydroxyalkylamino, cycloalkyl, halogen, haloalkyl, haloalkyloxy, NO₂, CN, SO₂NR⁴R⁵, CO₂NR⁴R⁵, COR⁴, CO₂R⁴, SO₂R⁴, SO₃R⁴, NR⁴R⁵, alkyl, aryl or heteroaryl;
- R^{3"}: is independently H, OR⁴, SR⁴, hydroxyalkyl, hydroxyalkylamino, cycloalkyl, halogen, haloalkyl, haloalkyloxy, NO₂, CN, SO₂NR⁴R⁵, CO₂NR⁴R⁵, COR⁴, CO₂R⁴, SO₂R⁴, SO₃R⁴, NR⁴R⁵, alkyl, aryl or heteroaryl;
- R⁴: is H, alkyl, cycloalkyl, aryl or heteroaryl;
- R⁵: is H, O-alkyl, O-aryl, alkyl, heteroaryl or aryl;
- Y¹ and Y²: are C=O ;
p is 0, m is 1, n is 1;
In Formula (I) the following definitions are used:
an alkyl group, if not stated otherwise, denotes a linear or branched C₁-C₆ alkyl, preferably a linear or branched chain of one to five carbon atoms, a linear or branched C₁-C₆-alkyl or a linear or branched C₁-C₆-alkinyl group, which can optionally be substituted by one or more substituents R³, preferably by halogen;
the C₁-C₄-alkyl, C₁-C₆-alkenyl and C₁-C₆-alkinyl residue may be selected from the group comprising -CH₃, -C₂H₅, -CH=CH₂, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R³)₃, -CR³(R^{3'})₂, -CR³(R^{3'})R^{3''}, -C₂(R³)₅, -CH₂-C(R³)₃, -CH₂-CR³(R^{3'})₂, -CH₂-CR³(R^{3'})R^{3''}, -C₃(R³)₇, -C₂H₄-C(R³)₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C₂H₅, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=(CH₃)-CH=CH₂, -CH=C(CH₃)=CH₂, -CH₂-CH=C(CH₃)₂, -C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C-C≡C-CH₃, -C≡C-CH₂-CH=CH₂, -CH=CH-CH₂C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, - C₃H₆-C≡C-CH₃, -CH₂-C≡C-C₃H₇, -C₂H₄-C≡C-C₂H₅;
a cycloalkyl group denotes a non-aromatic ring system containing three to eight carbon atoms, preferably four to eight carbon atoms, wherein one or more of the carbon atoms in the ring can be substituted by a group X, X being as defined above; the C₃-C₈-cycloalkyl residue may be selected from the group comprising -cyclo-C₃H₅, -cyclo-C₄H₇, -cyclo-C₅H₉, -cyclo-C₆H₁₁, -cyclo-C₇H₁₃, -cyclo-C₈H₁₅;
an alkoxy group denotes an O-alkyl group, the alkyl group being as defined above; the alkoxy group is preferably a methoxy, ethoxy, isopropoxy, t-butoxy or pentoxy group;
an haloalkyl group denotes an alkyl group which is substituted by one to five halogen atoms, the alkyl group being as defined above; the haloalkyl group is preferably a -C(R¹⁰)₃, -CR¹⁰(R^{10'})₂, -CR¹⁰(R¹⁰)R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇ or -C₂H₄-C(R¹⁰)₃, wherein R¹⁰, R^{10'}, R^{10"} represent F, Cl, Br or I, preferably F;
a hydroxyalkyl group denotes an HO-alkyl group, the alkyl group being as defined above;
an haloalkyloxy group denotes an alkoxy group which is substituted by one to five halogen atoms, the alkyl group being as defined above; the haloalkyloxy group is preferably a -OC(R¹⁰)₃, -OCR¹⁰(R^{10'})₂, -OCR¹⁰(R^{10'})R^{10"}, -OC₂(R¹⁰)₅, -OCH₂-C(R¹⁰)₃, -OCH₂-CR¹⁰(R^{10'})₂, -OCH₂-CR¹⁰(R^{10'})R^{10"}, -OC₃(R¹⁰)₇ or -OC₂H₄-C(R¹⁰)₃, wherein R¹⁰, R^{10'}, R^{10"} represent F, Cl, Br or I, preferably F;
a hydroxyalkylamino group denotes an (HO-alkyl)₂-N- group or HO-alkyl-NH- group, the alkyl group being as defined above;
a halogen group is chlorine, bromine, fluorine or iodine;
an aryl group preferably denotes an aromatic group having five to fifteen carbon atoms, which can optionally be substituted by one or more substituents R³, where R³ is as defined above; the aryl group is preferably a phenyl group, -CH₂Ph, -C₂H₄Ph, -CH=CH-Ph, -C≡C-Ph, -o-C₆H₄-R³, -m-C₆H₄-R³, -p-C₆H₄-R³, -o-CH₂-C₆H₄-R³, -m-CH₂-C₆H₄-R³, -p-CH₂-C₆H₄-R³;
a heteroaryl group denotes a 5- or 6-membered heterocyclic group which contains at least one heteroatom like O, N, S. This heterocyclic group can be fused to another ring. For example, this group can be selected from an oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, 1,2,4-oxadiazol-3-yl, 1,2,4-oxadiazol-5-yl, 1,2,4-thiadiazol-3-yl, 1,2,4-thiadiazol-5-yl, 1,2,5-oxadiazol-3-yl, 1,2,5-oxadiazol-4-yl, 1,2,5-thiad'iazol-3-yl, 1-imidazolyl, 2-imidazolyl, 1,2,5-thiadiazol-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, indolinyl, benzo-[b]-furanyl, benzo[b]thiophenyl, benzimidazolyl, benzothiazolyl, quinazolinyl, quinoxazolinyl, or preferably isoxazol-3-yl, isoxazol-4-yl, isoxazol-5-yl, quinolinyl, tetrahydroquinolinyl, isoquinolinyl, tetrahydroisoquinolinyl group. This heterocyclic group can optionally be substituted by one or more substituents R³, where R³ is as defined above.
In Formula (I), A¹ or A² each independently represent a C₁-C₂₀-alkyl group which is optionally substituted by one or more substituents R³, or a monocyclic or polycyclic aromatic or non-aromatic ring system which is optionally substituted by one or more substituents R³ and in case of an aromatic ring system contains at least one aromatic ring. The optionally substituted monocyclic or polycyclic aromatic or non-aromatic ring system may also contain one or more groups X selected from S, O, N, NR⁴, SO or SO₂. In preferred embodiments, A¹ and A² each independently represent an optionally substituted C₁-C₂₀-alkyl group or an optionally substituted monocyclic or bicyclic aromatic ring system. In case of substitutions of carbon atoms in the ring system, preferably one, two or three carbon atoms are substituted by a group X, wherein X is selected from the group consisting of S, O, N, NR⁴, SO or SO₂. In one preferred embodiment, one of the carbon atoms is substituted by a group X = O, S, NH.
In Formula (I), A¹ and/or A² independently represent an optionally substituted C₁-C₂₀-alkyl group which is optionally substituted by one or more substituents R³. Preferably A¹ and/or A² independently represent an optionally substituted C₁-C₁₂-alkyl group, said alkyl group may be a straight chain or branched chain alkyl group, and examples include methyl, ethyl, propyl, isopropyl, butyl, t-butyl, isobutyl, pentyl, hexyl, heptyl, octyl, nonyl, decyl, undecyl and dodecyl groups. The term alkyl group also contains alkenyl and alkinyl groups, that means that the alkyl group contains one or more double or triple bounds.
In Formula (I), A¹ and/or A² represent an optionally aromatic or non-aromatic ring system, which is substituted by one or more substituents R³, said ring system may be a phenyl, 1-naphthyl, 2-napthyl, 1-anthracenyl; 2-anthracenyl, 2-pyranyl, 3-pyranyl, 4-pyranyl, 2-thiazolyl, 4-thiazolyl, 5-thiazolyl, 2-oxazolyl, 4-oxazolyl and 5-oxazolyl, in particular 3-pyrazolyl, 4-pyrazolyl, 5-pyrazolyl, 2-pyrazinyl, 3-pyrazinyl, 1-imidazolyl, 2-imidazolyl, 2-thienyl, 3-thienyl, 2-furyl, 3-furyl, 2-pyridyl, 3-pyridyl, 4-pyridyl, benzothiophene, pyrazolo[3,4-b]-pyridyl, 2-pyrimidyl, 4-pyrimidyl and 9H-thioxanthene-10,10-dioxide ring, in which the ring system can be fused to one or more other monocyclic aromatic or non-aromatic rings.
Suitable substituents for A¹ and/or A² are independently H, NO₂, CN, CO₂R⁴, COR⁴, CONR⁴R⁵, NR⁴R⁵, OR⁴, SR⁴, hydroxyalkylamino, hydroxylalkyl, halogen, haloalkyl, haloalkyloxy, SO₂NR⁴R⁵, CO₂NR⁴R⁵, CO₂R⁴, SO₂R⁴, SO₃R⁴, NR⁴R⁵, alkyl, cycloalkyl, arylalkyl, aryl or heteroaryl.
Furthermore the present invention is directed to novel compounds of the general Formula (X) and pharmaceutically acceptable salts thereof: 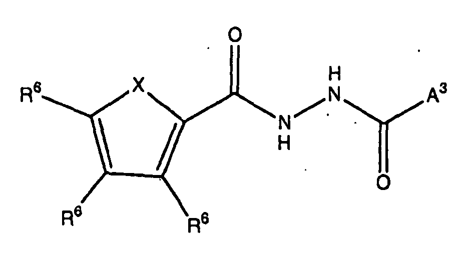 wherein
- A³: is an optionally substitued C₈-C₂₀-alkyl group or 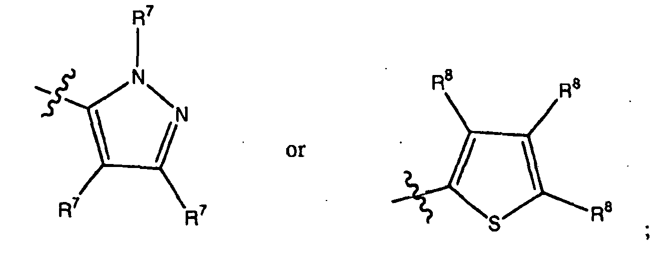 an C₈-C₂₀-alkyl group denotes a linear or branched C₈-C₂₀-alkyl group, which is optionally substituted by R³, R³ being as defined below; the C₈-C₂₀-alkyl residue may be selected from the group comprising -C₈H₁₇, -C₄H₈-C(CH₃)₃, -C₅H₁₀-CH(CH₃)₂, -C₄H₈-CH(CH₃)-C₂H₅, -C₃H₆-C(CH₃)₂-C₂H₅, -C₃H₆-CH(CH₃)-C₃H₇, -C₂H₄-C(CH₃)₂-C₃H₇, -C₂H₄-CH(CH₃)-C₄H₉, -CH₂-C(CH₃)₂-C₄H₉, -CH₂-CH(CH₃)-C₅H₁₁, -C(CH₃)₂-C₅H₁₁, -CH(CH₃)-C₆H₁₃, -C₉H₁₉, -C₅H₁₀-C(CH₃)₃, -C₆H₁₂-CH(CH₃)₂, -C₅H₁₀-CH(CH₃)-C₂H₅, -C₄H₈-C(CH₃)₂-C₂H₅, -C₄H₈-CH(CH₃)-C₃H₇, -C₃H₆-C(CH₃)₂-C₃H₇, -C₃H₆-CH(CH₃)-C₄H₉, -C₂H₄-C(CH₃)₂-C₄H₉, -C₂H₄-CH(CH₃)-C₅H₁₁, -CH₂-C(CH₃)₂-C₅H₁₁, -CH₂-CH(CH₃)-C₆H₁₃, -C(CH₃)₂-C₆H₁₃, -CH(CH₃)-C₇H₁₁, -C₁₀H₂₁, -C₆H₁₂-C(CH₃)₃, -C₇H₁₄-CH(CH₃)₂, -C₆H₁₂-CH(CH₃)-C₂H₅, -C₅H₁₀-C(CH₃)₂-C₂H₅, -C₅H₁₀-CH(CH₃)-C₃H₇, -C₄H₈-C(CH₃)₂-C₃H₇, -C₄H₈-CH(CH₃)-C₄H₉, -C₃H₆-C(CH₃)₂-C₄H₉, -C₃H₆-CH(CH₃)-C₅H₁₁, -C₂H₄-C(CH₃)₂-C₅H₁₁, -C₂H₄-CH(CH₃)-C₆H₁₃, -CH₂-C(CH₃)₂-C₆H₁₃, -CH₂-CH(CH₃)-C₇H₁₅, -C(CH₃)₂-C₇H₁₅, -CH(CH₃)-C₈H₁₇, -C₁₁H₂₃, -C₇H₁₄-C(CH₃)₃, -C₈H₁₆-CH(CH₃)₂, -C₇H₁₄-CH(CH₃)-C₂H₅, -C₆H₁₂-C(CH₃)₂-C₂H₅, -C₆H₁₂-CH(CH₃)-C₃H₇, -C₅H₁₀-C(CH₃)₂-C₃H₇, -C₅H₁₀-CH(CH₃)-C₄H₉, -C₄H₈-C(CH₃)₂-C₄H₉, -C₄H₈-CH(CH₃)-C₅H₁₁, -C₃H₆-C(CH₃)₂-C₅H₁₁, -C₃H₆-CH(CH₃)-C₆H₁₃, -C₂H₄-C(CH₃)₂-C₆H₁₃, -C₂H₄-CH(CH₃)-C₇H₁₅, -CH₂-C(CH₃)₂-C₇H₁₅, -CH₂-CH(CH₃)-C₈H₁₇, -C(CH₃)₂-C₈H₁₇, -CH(CH₃)-C₉H₁₉, -C₁₂H₂₅, -C₈H₁₆-C(CH₃)₃, -C₉H₁₈-CH(CH₃)₂, -C₈H₁₆-CH(CH₃)-C₂H₅, -C₇H₁₄-C(CH₃)₂-C₂H₅, -C₇H₁₄-CH(CH₃)-C₃H₇, -C₆H₁₂-C(CH₃)₂-C₃H₇, -C₆H₁₂-CH(CH₃)-C₄H₉, -C₅H₁₀-C(CH₃)₂-C₄H₉, - C₅H₁₀-CH(CH₃)-C₅H₁₁, -C₄H₈-C(CH₃)₂-C₅H₁₁, -C₄H₈-CH(CH₃)-C₆H₁₃, -C₃H₆-C(CH₃)₂-C₆H₁₃, -C₃H₆-CH(CH₃)-C₇H₁₅, C₂H₄-C(CH₃)₂-C₇H₁₅, -C₂H₄-CH(CH₃)-C₈H₁₇, CH₂-C(CH₃)₂-C₈H₁₇, -CH₂-CH(CH₃)-C₉H₁₉, -C(CH₃)₂-C₉H₁₉, -CH(CH₃)-C₁₀H₂₁;
- R⁶: is independently of each other -H, -F, -Cl, -Br, -I, -NO₂, -NR⁴R⁵, -CN, alkyl, cycloalkyl, -OH, alkoxy, alkylthio, hydroxyalkylamino, haloalkyl, haloalkyloxy, hydroxyalkyl, aryl or heteroaryl;
- R⁷: is independently of each other -H, -F, -Cl, -Br, -I, -NO₂, -NR⁴R⁵, -CN, alkyl, cycloalkyl, -OH, alkoxy, alkylthio, hydroxyalkylamino, haloalkyl, haloalkyloxy, hydroxyalkyl, aryl or heteroaryl;
- R⁸: is independently of each other -H, -F, -Cl, -Br, -I, -NO₂, -NR⁴R⁵, -CN, alkyl, cycloalkyl, -OH, alkoxy, alkylthio, hydroxyalkylamino, haloalkyl, haloalkyloxy, hydroxyalkyl, aryl or heteroaryl;
- X: is selected from the group consisting of S, O, N, NR⁴, SO or SO₂;
- R⁴: is H, alkyl, cycloalkyl, aryl or heteroaryl;
- R⁵: is H, O-alkyl, O-aryl, alkyl, heteroaryl or aryl;
R³, R^{3'} or R^{3"} are independently H, OR⁴, SR⁴, hydroxyalkyl, hydroxyalkylamino, cycloalkyl, halogen, haloalkyl, haloalkyloxy, NO₂, CN, SO₂NR⁴R⁵, CO₂NR⁴R⁵, COR⁴, CO₂R⁴, SO₂R⁴, SO₃R⁴, NR⁴R⁵, alkyl, aryl or heteroaryl; with R⁴, R⁵ being as defined above;
an alkyl group, if not stated otherwise, denotes a linear or branched C₁-C₆-alkyl, preferably a linear or branched chain of one to five carbon atoms, a linear or branched C₁-C₆-alkenyl or a linear or branched C₁-C₆-alkinyl group, which can optionally be substituted by one or more substituents R³, preferably by halogen;
the C₁-C₆-alkyl, C₁-C₆-alkenyl and C₁-C₆-alkinyl residue may be selected from the group comprising -CH₃, -C₂H₅, -CH=CH₂, -C≡CH, -C₃F₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R³)₃, -CR³(R^{3'})₂, -CR³(R^{3'})R^{3"}, -C₂(R³)₅, -CH₂- C(R³)₃, -CH₂-CR³(R^{3'})₂, -CH₂-CR³(R^{3'})R^{3''}, -C₃(R³)₇, -C₂H₄-C(R³)₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃; -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH₋C(CH₃)=CH₂, -CH₂-CH=C(CH₃)₂, -C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C≡CH, -CH₂-C≡C-C≡CH, -C≡C-CH=CH-CH₃, -CH=CH-C≡C-CH₃, -C≡C=C-CH₃, -C≡C-CH₂-CH=CH₂, -CH=CH--CH₂-C≡CH, -C≡C-CH₂CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C=CH, -C=C-C(CH₃)=CH₂, C₃H₆-CH(CH₃)_{2,} -C₂H₁₄-CH(CH₃)-C₂H₅, -CH(CH₃)-C₄H₉, -CH₂-CH(CH₃)-C₃H₇, -CH(CH₃)CH₂-CH(CH₃)₂,-CH(CH₃)-CH(CH₃)₂H₅; -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-(CH₃)₃, -C₄H₈-CH=CH₂, -CH=CH-C₄H₉, -C₃-H₆=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, - C₃H₆-C≡C-CH₃, -CH₂-C≡C-C₃H₇, -C₂H₄-C≡C₂H₅;
a cycloalkyl group denotes a non-aromatic ring system containing three to eight carbon atoms, preferably four to eight carbon atoms, wherein one or more of the carbon atoms in the ring can be substituted by a group X, X being as defined above; the C₃-C₈-cycloalkyl residue may be selected from the group comprising -cyclo-C₃H₅, -cyclo-C₄H₇, -cyclo-C₅H₉, -cyclo-C₆H₁₁, -cyclo-C₇H₁₃, -cyclo-C₈H₁₅;
an alkoxy group denotes an O-alkyl group, the alkyl group being as defined above; the alkoxy group is preferably a methoxy, ethoxy, isopropoxy, *t*-butoxy or pentoxy group.
an haloalkyl group denotes an alkyl group which is substituted by one to five halogen atoms, the alkyl group being as defined above; the haloalkyl group is preferably a -C(R¹⁰)₃, -CR¹⁰(R^{10'})₂, -CR¹⁰(R^{10'})R^{10"}, -C₂(R¹⁰)₅, -CH₂-C(R¹⁰)₃, -CH₂-CR¹⁰(R^{10'})₂, -CH₂-CR¹⁰(R^{10'})R^{10"}, -C₃(R¹⁰)₇ or -C₂H₄-C(R¹⁰)₃, wherein R¹⁰, R^{10'}, R^{10"} represent F, Cl, Br or I, preferably F;
a hydroxyalkyl group denotes an HO-alkyl group, the alkyl group being as defined above;
an haloalkyloxy group denotes an alkoxy group which is substituted by one to five halogen atoms, the alkyl group being as defined above; the haloalkyloxy group is preferably a -OC(R¹⁰)₃, -OCR¹⁰(R^{10'})₂, -OCR¹⁰(R^{10'})R^{10"}, -OC₂(R¹⁰)₅, -OCH₂-C(R¹⁰)₃, -OCH₂-CR¹⁰(R^{10'})₂, -OCH₂-CR¹⁰(R^{10'})R^{10"}, -OC₃(R¹⁰)₇ or -OC₂H₄-C(R¹⁰)₃, wherein R¹⁰, R^{10'}, R^{10"} represent F, Cl, Br or I, preferably F;
a hydroxyalkylamino group denotes an (HO-alkyl)₂-N- group or HO-alkyl-NH- group, the alkyl group being as defined above;
a halogen group is chlorine, bromine, fluorine or iodine;
an aryl group preferably denotes an aromatic group having five to fifteen carbon atoms, which can optionally be substituted by one or more substituents R³, where R³ is as defined above; the aryl group is preferably a phenyl group, -CH₂Ph, -C₂H₄Ph, -CH=CH-Ph, -C≡C-Ph, -o-C₆H₄-R³, -m-C₆H₄-R³, -p-C₆H₄-R³, -o-CH₂-C₆H₄-R³, -m-CH₂C₆H₄-R³, -p-CH₂-C₆H₄-R³;
a heteroaryl group denotes a 5- or 6-membered heterocyclic group which contains at least one heteroatom like O, N, S. This heterocyclic group can be fused to another ring. For example, this group can be selected from an oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, 1,2,4-oxadiazol-3-yl, 1,2,4-oxadiazol-5-yl, 1,2,4-thiadiazol-3-yl, 1,2,4-thiadiazol-5-yl, 1,2,5-oxadiazol-3-yl, 1,2,5-oxadiazol-4-yl, 1,2,5-thiadiazol-3-yl, 1-imidazolyl, 2-imidazolyl, 1,2,5-thiadiazol-4-yl, 4-imidazolyl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, indolinyl, benzo-[b]-furanyl, benzo[b]thiophenyl, benzimidazolyl, benzothiazolyl, quinazolinyl, quinoxazolinyl, or preferably isoxazol-3-yl, isoxazol-4-yl, isoxazol-5-yl, quinolinyl, tetrahydroquinolinyl, isoquinolinyl, tetrahydroisoquinolinyl group. This heterocyclic group can optionally be substituted by one or more substituents R³, when R³ is as defined above.

However, the following compounds are excluded from Formula (X): 2-thiophenecarboxylic acid-5-nitro-2-(2-thienylcarbonyl) hydrazide, 4-butylthiophene-2-carboxylic acid-*N*'-(4-butyl-tiophen-2-carbonyl) hydrazide, 2-thiophene-carboxylic acid-3-chloro-2-(2-thienylcarbonyl) hydrazide, 2-thiophene carboxylic acid-5-bromo-2-(2-thienylcarbonyl) hydrazide, 1H-pyrazole-5-carboxylic acid-1-methyl-2-(2-thienylcarbonyl) hydrazide, 2-thiophenecarboxylic acid-5-(4,5,6,7-tetrahydro-benzo[b]thien-2-yl)-2-[[5-(4,5,6,7-tetrahydrobenzo[b]thien-2-yl)-2-thienyl]-carbonyl]-hydrazide, 1H-pyrrole-2-carboxylic acid-2-(2-thienylcarbonyl)hydrazide, 2-thiophenecarboxylic acid-2-(2-thienylcarbonyl)-hydrazide, 2-thiophenecarboxylic acid *N'*-(furan-2-carbonyl) hydrazide, 2-thiophenecarboxylic acid-*N'*-(5-bromofuran-2-carbonyl) hydrazide, 1H-pyrazole-3-carboxylic acid-4-bromo-1,5-dimethyl-2-(2-thienylcarbonyl)hydrazide, thiophene-2-caboxylic acid *N'*-(3-chloro-4-methylthiophene-2-carbonyl) hydrazide and 2-furancarboxylic acid-5-[[[4-methyl-6-(trifluoromethyl)-2-pyrimidinyl]thio]methyl]-2-(2-thienylcarbonyl)hydrazide.

Furthermore the present invention is detected to novel compounds of the general Formula (X) and pharmaceutically acceptable salts thereof: 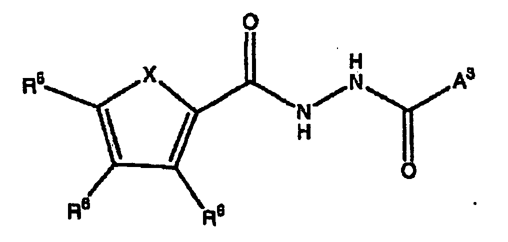 wherein
- A³: is an C₈-C₂₀-alkyl group or 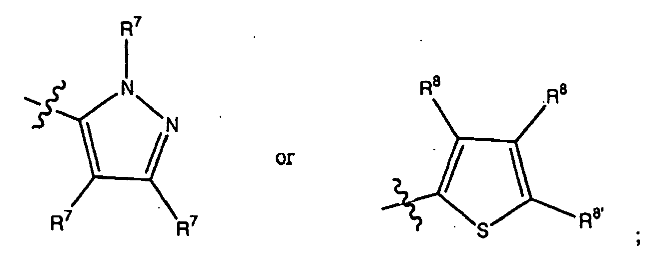
with the C₈-C₂₀-alkyl group being as defined above for Formula (X)
- R³: is defined as above in Formula (X)
- R⁴: is defined as above in Formula (X)
- R⁵: is defined as above in Formula (X)
- R⁶: is independently of each other -H, -F, -Cl, -Br, -I, -NO₂, -NR⁴R⁵, -CN, C₁-C₃-alkyl, -OH, alkoxy, alkylthio, hydroxyalkylamino, haloalkyl, haloalkyloxy, hydroxyalkyl, aryl or heteroaryl;
said C₁-C₃-alkyl of R⁶ denotes a linear or branched C₁-C₃-alkyl, a linear or branched C₁-C₃-alkenyl or a linear or branched C₁-C₃-alkinyl group, which can optionally be substituted by one or more substituents R', preferably by halogen; the C₁-C₃-alkyl, C₁-C₃-alkenyl and C₁-C₃-alkinyl residue may be selected from the group comprising -CH₃, -C₂H₅, -CH=CH₂, -C≡CH, -C₃H₇, -CH(CH₃)₂, -C≡C-CH₃, -CH₂-C≡CH;
R is independently H, OR⁴, hydroxyalkyl, hydroxyalkylamino, cycloalkyl, halogen, haloalkyl, haloalkyloxy, NO₂, CN, SO₂NR⁴R⁵, CO₂NR⁴R⁵, COR⁴, CO₂R⁴, SO₂R⁴, SO₃R⁴, NR⁴R⁵, alkyl, aryl or heteroaryl;
- R¹: is independently of each other -H, -F, -Cl, -I, -NO₂, -NR⁴R⁵, -CN, C₂-C₆-alkyl, cycloalkyl, -OH, alkoxy, alkylthio, hydroxyalkylamino, haloalkyl, haloalkyloxy, hydroxyalkyl, aryl or heteroaryl;
said C₂-C₆-alkyl of R⁷, denotes a linear or branched C₂-C₆-alkyl, a linear or branched C₂-C₆-alkenyl or a linear or branched C₂-C₆-alkinyl group, which can optionally be substituted by one or more substituents R³, preferably by halogen; the C₂-C₆-alkyl, C₂-C₆-alkenyl and C₂-C₄-alkinyl residue may be selected from the group comprising -C₂H₅, -CH=CH₂, -C≡CH, -C₃H₇, -CH(CH₃)₂, -C≡C-CH₃, -CH₂-C≡CH, -CH₂-CH=CH₂, -C(CH₃)=CH₂, -CH=CH-CH₃, -C₄H₉, -CH₂-CH(CH₃)₂, -CH(CH₃)-C₂H₅, -C(CH₃)₃, -C₅H₁₁, -C₆H₁₃, -C(R³)₃, -CR³(R^{3'})₂, -CR³(R^{3'})R^{3"}, -C₂(R³)₅, -CH₂-C(R)³, -CH₂-CR³(R^{3'})₂, -CH₂-CR³(R^{3'})R^{3"}, -C₃(R³)₇, -C₂H₄-C(R³)₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₃H₆-C≡CH, -C≡C-C₃H₇, -C₂H₄-C≡C-CH₃, -CH₂-C≡C-C₂H₅, -CH₂-C≡C-CH=CH₂, -CH₂-CH=CH-C=CH, -CH₂-C≡C-C=CH, -C≡C-CH=CH-CH₃, -CH=CH-C≡C-CH₃, -C≡C-C≡C-CH₃, -C≡C-CH₂-CH=CH₂, -CH=CH-CH₂-C=CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C=CH, -CH=C(CH₃)-C≡CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅; R³, R^{3'} or R^{3"} are defined as above in Formula (X) R⁴, R⁵ are defined as above in Formula (X);
- R⁸: is independently of each other -H, -F, -I, -NR⁴R⁵, -CN, C₁-C₃-alkyl, -OH, alkoxy, alkylthio, hydroxyalkylamino, haloalkyl, haloalkyloxy, hydroxyalkyl, aryl or heteroaryl;
said C₁-C₃-alkyl of R⁸, denotes a linear or branched C₁-C₃-alkyl, a linear or branched C₁-C₃-alkenyl or a linear or branched C₁-C₃-alkinyl group, which can optionally be substituted by one or more substituents R', preferably by halogen; the C₁-C₃-alkyl, C₁-C₃-alkenyl and C₁-C₃-alkinyl residue may be selected from the group comprising -CH₃, -C₂H₅, -CH=CH₂, C≡CH, -C₃H₇, -CH(CH₃)₂, -C≡C-CH₃, -CH₂C≡CH;
R is defined as above in Formula (X);
R⁴, R⁵ are defined as above in Formula (X);
- R^{8'}: is independently of each other -F, -I, -NR⁴R⁵, -CN, C₁-C₃-alkyl, -OH, alkoxy,
alkylthio, hydroxyalkylamino, haloalkyl, haloalkyloxy, hydroxyalkyl, aryl or heteroaryl;
said C₁-C₃-alkyl of R^{8'} denotes a linear or branched C₁-C₃-alkyl, a linear or branched C₁-C₃-alkenyl or a linear or branched C₁-C₃-alkinyl group, which can optionally be substituted by one or more substituents R', preferably by halogen; the C₁-C₃-alkyl, C₁-C₃-alkenyl and C₁-C₃-alkinyl residue may be selected from the group comprising -CH₃, -C₂H₅, -CH=CH₂, -C≡CH, -C₃H₇, -CH(CH₃)₂, -C≡C-CH₃, -CH₂-C≡CH; R is defined as above in Formula (X);
an alkyl group, referring to R³, R^{3'} R^{3"}, R⁴ or R⁵ denotes a linear or branched C₁-C₆-alkyl, preferably a linear or branched chain of 1 to 5 carbon atoms, a linear or branched C₁-C₆-alkenyl or a linear or branched C₁-C₆-alkinyl group, which can optionally be substituted by one or more substituents R³, preferably by halogen;
the C₁-C₆-alkyl, C₁-C₆-alkenyl and C₁-C₆-alkinyl residue may be selected from the group comprising -CH₃, -C₂H₅, -CH=CH₂, -C≡CH, -C₃H₇, -CH(CH₃)₂, -CH₂-CH=CH₂, -C(CH₃)=CH₂, -CH=CH-CH₃, -C≡C-CH₃, -CH₂-C≡CH, -C₄H₉, -CH₂-CH(CH₃)₂, -CH(CH₃)-C₂H₅, -C(CH₃)₃, -C₅H₁₁, -C₆H₁₃, -C(R³)₃, -CR³(R^{3'})₂, -CR³(R^{3'})R^{3"}, -C₂(R³)₅, -CH₂-C(R³)₃, -CH₂-CR³(R^{3'})₂, -CH₂-CR³(R^{3'})R^{3"}, -C₃(R³)₇, -C₂H₄-C(R³)₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃, -C≡C-CH=CH₂, -CH=CH-C≡CH, -C≡C-C≡CH, -C₂H₄-CH(CH₃)₂, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -CH₂-CH=C(CH₃)₂, -C(CH₃)=C(CH₃)₂, -C₃H₆-C=CH, -C≡C-C₃H₇, -C₂H₄-C≡C-CH₃, -CH₂-C≡C-C₂H₅, -CH₂-C=C-CH=CH₂, -CH₂-CH=CH-C=CH, -CH₂-C≡C-C≡CH, -C=C-CH=CH-CH₃, -CH=CH-C≡C-CH₃, -C≡C-C≡C-CH₃, -C≡C-CH₂-CH=CH₂, -CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C=CH, -CH=C(CH₃)-C=CH, -C≡C-C(CH₃)=CH₂, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)=C(CH₃)₂, -C₂H₄-CH=C(CH₃)₂, -C₄H₈-C≡CH, -C≡C-C₄H₉, -C₃H₆-C≡C-CH₃, -CH₂-C≡C-C₃H₇, -C₂H₄-C≡C-C₂H₅;
R³, R^{3'} of R^{3"} are defined as above in Formula (X);
a cycloalkyl group is defined as above in Formula (X);
an alkoxy group is defined as above in Formula (X):
an haloalkyl is defined as above for in Formula (X):
a hydroxyalkyl group is defined as above in Formula (X);
an haloalkyloxy group is defined as above in Formula (X);
a hydroxyalkylamino group is defined as above in Formula (X);
a halogen group is defined as above in Formula (X);
an aryl group is defined as above in Formula (X);
a heteroaryl group is defined as above in Formula (X).

The invention also provides a pharmaceutical composition comprising a compound of Formula (I) or (X), in free form or in the form of pharmaceutically acceptable salts and physiologically functional derivatives, together with a pharmaceutically acceptable diluent or carrier therefore.

The term "physiologically functional derivative" as used herein refers to compounds which are not pharmaceutically active themselves but which are transformed into their pharmaceutical active form *in vivo,* i.e. in the subject to which the compound is administered.

In another aspect, the present invention also provides a method for the treatment or prophylaxis of a condition where there is an advantage in inhibiting quorum sensing which comprises the administration of an effective amount of a compound of Formula (I) or (X) and physiologically acceptable salts or physiologically functional derivatives thereof. The term "quorum sensing" is intended to describe cell-density dependent gene regulation through a diffusible signal molecule (Fuqua et al., J. Bacteriol. 176:269-75, 1994)

The invention is also directed to the use of compounds of Formula (I) or (X) and of their pharmacologically tolerable salts or physiologically functional derivatives for the production of a medicament or medical device for the prevention and treatment of diseases, where quorum sensing inhibition is beneficial. Furthermore, the invention is also directed to the use of compounds of Formula (I) or (X) and of their pharmacologically tolerable salts or physiologically functional derivatives for the production of an antibacterial agent for the prevention and treatment of bacterial biofilms in industrial and environmental settings.

In addition, the present invention provides methods for preparing the desired compounds of Formula (I) or (X)
One method for preparing the compounds of Formula (I) ( p = 0 / m, n =1) or compounds of Formula (X) comprises the step of reacting a compound of Formula (IV) with a compound of Formula (III). Other methods for preparing different 1,2-diacylhydrazines are described in Houben-Weyl, "Methoden der organischen Chemie", Vierte Auflage. G. Thieme Verlag, J. Falbe (ed), vol. E5, p. 1173-80 or P. A. S. Smith, "Open-Chain Organic Nitrogen Compounds", W. A Benjamin Inc., New York, vol. 2, p. 173-201. Methods for preparing different 1,2-disulfonylhydrazines are described in Arch. Pharm. 1953, 286, 338-43 or in US 6291504. Methods for preparing 1-acyl-2-sulfonylhydrazines are described in Russ. J. Gen. Chem. 2000, 70, 3, 459-60 or by Leadini et al., J. Chem. Soc. Perkin Trans. 1 1998, 1833-8 and by M. Reinecke et al., J. Org. Chem. 1988, 53, 1, 208-10.

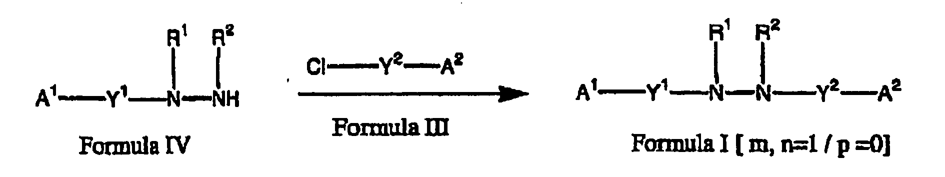

A more preferred compound of Formula (I) is a compound wherein p is 0 and m, n are 1, one of A¹ and A² represent an optionally substituted 5-membered aromatic ring system, and the other one of A¹ and A² represent an optionally substituted alkyl group -or a substituted monocyclic aromatic ring system.

A more preferred compound of Formula (I) is a compound wherein p is 0 and m, n are 1, A¹ and A² represent an optionally substituted 5-membered aromatic ring system.

In the compounds of Formula (I), R is H,

In the compounds of Formula (I), R¹ is H.

In the compounds of Formula (I), R² is H.

Preferably, R³ in Formula (I), or (X), is independently H, halogen, CF₃, OCF₃, phenyl or alkyl.

R⁴ in Formula (I), or (X) is independently H, alkyl, cycloakyl, aryl or heteroaryl. Preferably R⁴ is H.

R⁵ in Formula (I), or (X) is independently H, O-alkyl, O-aryl, alkyl; heteroaryl or aryl. Preferably R⁵ is H.

In Formula (I) Y¹ and Y² are CO.

In Formula (I) Z is independently S, O, N, NR⁴, CO, CO₂, CS, SO or SO₂. Preferably, Z is 0, CO, CO₂.

In Formula (I) or (X) X is independently S, O, N, NR⁴, SO or SO₂. Preferably, X is N, S, O, NR⁴.

Most preferred is the use of one or more compounds of Formula (I), or (X), including the compounds excluded by any of the disclaimers, and/or pharmaceutically acceptable salts thereof for regulation of the quorum sensing system of microorganisms, in particular gram-negative bacteria. In one embodiment of the invention also the use of the following compounds is preferred: 2-Methyl-6-trifluoromethyl-nicotinic acid N-(thiophene-2-carbonyl)-hydrazide; 4-Trifluoromethylbenzoic acid N'-(4-methyl-thiophene-2-carbonyl)-hydrazide; 4-Chloro-benzoic acid N'-(4-methoxy-thiophene-3-carbonyl)-hydrazide; 3-Chloro-benzoic acid N'-(thiophene-2-carbonyl)-hydrazide; N'-(3-Methyl-1,4-dioxy-quinoxaline-2-carbonyl)-thiophene-2-carboxylic acid hydrazide; Furan-2-carboxylic acid N'-(thiophene-2-carbonyl)-hydrazide; Furan-2-carboxylic acid N'-(3-chloro-4-methyl-thiophene-2-carbonyl)-hydrazide; Furan-2-carboxylic acid N'-(3-ethoxy-thiophene-2-carbonyl)-hydrazide; Furan-2-carboxylic acid N'-(3-chloro-benzo[b]thiophene-2-carbonyl)-hydrazide; Thiophene-2-carboxylic acid N'-(5-bromo-4-methoxy-thiophene-3-carbonyl)hydrazide; Thiophene-2-carboxylic acid N'-(3-chloro-4-methyl-thiophene-2-carbonyl)-hydrazide; Thiophene-2-carboxylic acid N'-(thiophene-2-carbonyl)-hydrazide; 3-Chloro-thiophene-2-carboxylic acid N'-(thiophene-2-carbonyl)-hydrazide; Thiophene-2-carboxylic acid N'(5-bromo-thiophene-2-carbonyl)-hydrazide; 3-Chloro-benzo[b]thiophene-2-carboxylic acid N'-(thiophene-2-carbonyl)-hydrazide; Thiophene-2-carboxylic acid N'-(4-bromo-1,5-dimethyl-1H-pyrazole-3-carbonyl)-hydrazide; Thiophene-2-carboxylic acid N'-butyryl-hydrazide.

Preferred compounds of the present invention and/or pharmaceutically acceptable salts thereof are selected from the group comprising: 4-Trifluoromethoxy-benzoic acid N'-(thiophene-2-carbonyl)-hydrazide; 3-Chloro-thiophene-2-carboxylic acid N-(1-phenyl-5-trifluoromethyl-1H-pyrazole-4-carbonyl)-hydrazide; Thio-phene-2-carboxylic acid N'-[1-(4-chloro-phenyl)-5-trifluoromethyl-1H-pyrazole-4-carbonyl]-hydrazide; Furan-2-carboxylic acid N'-(5-chloro-4-methoxy-thiophene-3-carbonyl)-hydrazide; Furan-2-carboxylic acid N'-(3-bromo-thiophene-2-carbonyl)-hydrazide; Furan-2-carboxylic acid N'-(2,5-dichloro-thiophene-3-carbonyl)-hydrazide; 3-Chloro-thiophene-2-carboxylic acid N'-(3-ethoxy thiophene-2-carbonyl)-hydrazide; Thiophene-2-carboxylic acid N'-(3-ethoxy-thiophene-2-carbonyl)-hydrazide; Thiophene-3-carboxylic acid N'-(thiophene-2-carbonyl)-hydrazide; 3-Chloro-thiophene-2-carboxylic acid N'-(3-chloro-thiophene-2-carbonyl)-hydrazide; 5-Chloro-4-methoxy-thiophene-3-carboxylic acid N'-(thiophene-2-carbonyl)-hydrazide; Thiophene-2-carboxylic acid N'-(5-chloro-thiophene-2-carbonyl)-hydrazide; 5-Bromo-4-methoxythiophene-3-carboxylic acid N-(5-methyl-thiophene-2-carbonyl)-hydrazide; Thiophene-2-carboxylic acid N'-(5-methyl-thiophene-2-carbonyl)-hydrazide; Thiophene-2-carboxylic acid N'-(3-bromo-thiophene-2-carbonyl)-hydrazide; 3-Bromo-thiophene-2-carboxylic acid N'-(3-ethoxy-thiophene-2-carbonyl)-hydrazide; 3-Bromo-thiophene-2-carboxylic acid N'-(5-methylthiophene-2-carbonyl)-hydrazide; 3-Chloro-thiophene-2-carboxylic acid N'-(5-chloro-thiophene-2-carbonyl)-hydrazide; 3-Chloro-benzo[b]thiophene-2-carboxylic acid N'-(3-bromo-thiophene-2-carbonyl)-hydrazide; 3-Chloro-benzo[b]thiophene-2-carboxylic acid N'-(5-bromo-thiophene-2-carbonyl)-hydrazide; 4-Chloro-1,3-dimethyl-1H-pyrazolo[3,4-b]-pyridine-5-carboxylic acid N'-(thiophene-2-carbonyl)-hydrazide; 2,5-Dimethyl-2H-pyrazole-3-carboxylic acid N'-(3-chloro-benzo[b]thiophene-2-carbonyl)-hydrazide; 2,5-Dimethyl-2H-pyrazole-3-carboxylic acid N'-(thiophene-2-carbonyl)-hydrazide; 4-Bromo-2-ethyl-5-methyl-2H-pyrazole-3-carboxylic acid N'-(5-chloro-thiophene-2-carbonyl)-hydrazide; Thiophene-2-carboxylic acid N'-(2-tert-butyl-5-methyl-2H-pyrazole-3-carbonyl)-hydrazide; 5-tert-Butyl-2-methyl-2H-pyrazole-3-carboxylic acid N'-(5-chloro-thiophene-2-carbonyl)-hydrazide; Thiophene-2-carboxylic acid N'-(5-tert-butyl-2-methyl-2H-pyrazole-3-carbonyl)-hydrazide; 4-Bromo-2-ethyl-5-methyl-2H-pyrazole-3-carboxylic acid N'-(5-bromo-thiophene-2-carbonyl)-hydrazide; 4-Bromo-2-ethyl-5-methyl-2H-pyrazole-3-carboxylic acid N'-(3-chloro-4-methylthiophene-2-carbonyl)-hydrazide; 4-Bromo-2-ethyl-5-methyl-2H-pyrazole-3-carboxylic acid N'-(5-chloro-thiophene-2-carbonyl)-hydrazide ; 4-Bromo-2-ethyl-5-methyl-2H-pyrazole-3-carboxylic acid N'-(4,5-di-bromo-thiophene-2-carbonyl)-hydrazide; 5-Methylthiophene-2-carboxylic acid N'-butyryl-hydrazide; 5-Bromo-thiophene-2-carboxylic acid N'-butyryl-hydrazide; Thiophene-2-carboxylic acid N'-(6-bromo-hexanoyl)-hydrazide; Thiophene-2-carboxylic acid N'-heptanoyl-hydrazide; 3-Chloro-4-methyl-thiophene-2-carboxylic acid N'-(6-bromo-hexanoyl)-hydrazide; 3-Chloro-4-methyl-thiophene-2-carboxylic acid N'-heptanoyl-hydrazide; 5-Methyl-thiophene-2-carboxylic acid N'-(6-bromo-hexanoyl)-hydrazide; 5-Methyl-thiophene-2-carboxylic acid N'-heptanoyl-hydrazide; 5-Bromothiophene-2-carboxylic acid N'-heptanoyl-hydrazide; 3-Bromo-thiophene-2-carboxylic acid N'-octanoyl-hydrazide; Thiophene-2-carboxylic acid N'-dodecanoyl-hydrazide; 5-Methylthiophene-2-carboxylic acid N'-(3-cyclopentyl-propionyl)-hydrazide; 5-[N'-(5-Methyl-thiophene-2-carbonyl)-hydrazino]-5-oxo-pentanoic acid methyl ester; Furan-2-carboxylic acid N'-heptanoyl-hydrazide; Furan-2-carboxylic acid N'-(3-cyclopentyl-propionyl)-hydrazide; 3,5-Dimethyl-isoxazole-4-carboxylic acid N'-octanoyl-hydrazide; 4-Bromo-2-ethyl-5-methyl-2H-pyrazole-3-carboxylic acid N'-octanoyl-hydrazide; 2-Chloro-isonicotinic acid N'-octanoyl-hydrazide; 2-Chloro-nicotinic acid N-octanoyl-hydrazide; 3-[N'-(3-Bromothiophene-2-carbonyl)hydrazino]-3-oxo-propionic acid ethyl ester; 3-[N'-(Benzo[b]thiophene-2-carbonyl)-hydrazino]-3-oxo-propionic acid ethyl ester; N'-(5-Chloro-thiophen-2-sulfonyl)-heptanoic acid hydrazide; 1-(4,5-Dibromo-thiophene-2-carbonyl)-4-pentyl-semicarbazide;
The compounds of the Formula (I), or (X) according to the invention can be also used in form of the corresponding salts with inorganic or organic acids or bases. Examples of such salts are, e.g., alkali metal salts, in particular sodium and potassium salts, or ammonium salts.
In general, the compounds of the present invention can be used to inhibit quorum sensing signaling of bacteria employing HSLs as signal molecules for cell-cell communication. Preferably, the compounds can be applied to the bacteria listed in Table 1, and more preferably to the bacteria of Table 1 that are pathogens. In the following it is explained that the compounds of the present invention can be used as antibacterial agents in various applications.

In a preferred form, the compounds of Formula (I), or (X), are useful for the treatment of a variety of human, animal and plant diseases, where bacterial pathogens regulate the expression of virulence genes and other phenotypes, e.g. biofilm formation, through an HSL-based quorum sensing system. Furthermore, as the list of organisms (see Table 1) employing quorum sensing signaling for their virulence continues to increase, the compounds of the invention can be used also for organisms which will be added to the above listed in future.

In a first embodiment, the compounds are useful for the treatment of mammalian in particular human diseases caused by bacteria through the inhibition of the bacterial quorum sensing cascade rendering the pathogen avirulent. Such diseases include endocarditis, respiratory and pulmonary infections (preferably in immunocompromized and cystic fibrosis patients), bacteremia, central nervous system infections, ear infections including external otitis, eye infections, bone and joint infections, urinary tract infections, gastrointestinal infections and skin and soft tissue infections including wound infections, pyoderma and dermatitis which all can be triggered by *Pseudomonas aeruginosa.* Furthermore, the compounds can be used for the treatment of pulmonary infections caused by *Burkholderia cepacia* (preferably in immunocompromized and cystic fibrosis patients), gastroenteritis and wound infections caused by *Aeromonas hydrophila,* sepsis in tropical and subtropical areas caused by *Chromobacterium violaceum,* diarrhoea with blood and haemolytic uremia syndrome (HUS) caused by *Escherichia coli,* yersiniosis triggered by *Yersinia enterocolitica* and *Y. pseudotuberculosis,* and transfusion-related sepsis and fistulous pyoderma caused by *Serratia liquefaciens.*

In a second embodiment, the compounds can be used to prevent and/or treat plant diseases, where inhibition of the HSL-mediated signaling system reduces or abolishes virulence of bacterial plant pathogens. Such diseases include crown gall tumors caused by *Agrobacterium tumefaciens,* soft rot caused by *Burkholderia cepacia, Erwinia carotovora* and *Erwinia chrysanthemi,* sweet corn and maize infections caused by *Pantoea stewartii* and wilt disease caused by *Ralstonia solanacearum.*

In a third embodiment, the compounds can be used for the prevention and/or treatment of animal diseases, preferably fish diseases such as septicemia caused by *Aeromonas hydrophila* and *Vibrio anguillarum,* furunculosis in salmonids caused by *Aeromonas salmonicida,* prawn infections caused by *Vibrio harveyi* and enteric redmouth disease caused by *Yersinia ruckeri,* but also for the prevention and/or treatment of insect diseases caused, for example, by *Xenorhabdus nematophilus.*
In general, the present invention provides a method for reducing the virulence of bacterial pathogens employing an HSL-based signaling system. In a preferred form, a method is provided to remove, diminish, detach or disperse a bacterial biofilm from a living or nonliving surface by treating the surface with a compound of Formula (I), or (X). This method is also useful to prevent biofilm formation on a living or nonliving surface by treating the surface with a compound of Formula (I), or (X), before bacterial colonization can initialize. The term "biofilm" refers to cell aggregations comprising either a single type of organism or a mixture of more than one organism, then also referred to as "mixed biofilms". It is clear to persons skilled in the art, that the compounds of the present invention can be applied in a wide variety of different fields such as environmental, industrial and medical applications in order to prevent and/or treat damages or diseases caused by bacteria.

In one aspect, the compounds of Formula (I), or (X), can be used for all kinds of surfaces in private and public areas, where it is beneficial to inhibit quorum sensing systems of Gram-negative bacteria in order to prevent and/or treat colonization and biofilm formation. The compounds here can be used in form of a solution, powder or as a coating. The compound is preferably applied to the surface as a solution of the compound, alone or together with other materials such as conventional surfactants, preferably sodium dodecyl sulfate, or detergents, biocides, fungicides, antibiotics, pH regulators, perfumes, dyes or colorants. In combination with a bacteriocidal agent, e.g., the compounds of Formula (I), or (X), inhibit virulence or biofilm formation whilst the bacteriocidal agent kills the pathogens.

In one embodiment, the compounds can be used as antibacterial agent for topical use in cleaning and treatment solutions such as disinfectants, detergents, household cleaner and washing powder formulations in the form of a spray or a dispensable liquid. In a preferred form, these solutions can be applied to windows, floors, clothes, kitchen and bathroom surfaces and other surfaces in the area of food preparation and personal hygiene. In addition, the compounds of Formula (I), or (X), can be used as antibacterial ingredients in personal hygiene articles, toiletries and cosmetics such as dentifrices, mouthwashes, soaps, shampoos, shower gels, ointments, creams, lotions, deodorants and disinfectants and storage solutions for contact lenses. In the case of contact lenses the compounds of Formula (I), or (X), can also be applied as coating or additive to the lens material.

In another embodiment, the compounds can be used to prevent or treat bacterial biofilms in industrial settings such as ship hulls, paper and metal manufacturing, oil recovery, food processing and other applications where process disturbances are referred to biofouling on surfaces. The compounds here can be used in form of a solution, paint or coating, for example as an ingredient in cooling lubricants. The compounds can also be applied to water processing plants or drinking water distribution systems where the colonized surface (preferably by *Pseudomonas aeruginosa*) is preferably the inside of an aqueous liquid system such as water pipes, water injection jets, heat exchangers and cooling towers. Until now biocides are the preferred tools to encounter these problems, but since biocides do not have a high specificity for bacteria, they are often toxic to humans as well This can be circumvented by the application of the compounds of the present invention.

In a further embodiment, the present invention relates to a method of inhibiting and/or preventing medical device-associated bacterial infections. The invention provides articles coated and/or impregnated with a compound of Formula (I), or (X) in order to inhibit and/or prevent biofilm formation thereon. The articles are preferably surgical instruments, blood bag systems or medical devices; more preferably either permanently implanted devices such as artificial heart valve, prostethic joint, voice prosthesis, stent, shunt or not permanently implanted devices such as endotracheal or gastrointestinal tube, pacemaker, surgical pin or indwelling catheter.

In a more preferred form, the indwelling catheters are urinary catheters, vascular catheters, peritoneal dialysis catheter, central venous catheters and needleless connectors. The catheter materials can be polyvinylchloride, polyethylene, latex, teflon or similar polymeric materials, but preferably polyurethane and silicone or a mixture thereof. In order to reduce the risk of catheter-related bacterial infections, several catheters coated and/or impregnated with antiseptic or antimicrobial agents such as chlorhexidine/silver-sulfadiazine and minocycline/rifampin, respectively, have been developed. Furthermore, collection bags or layers sandwiched between an external surface sheath and a luminal silicone sheath have been constructed to overcome rapid loss of antimicrobial activity. Nevertheless, the emerging risk of bacterial resistance against traditional antibiotics limits the routine use of antibiotic-coated catheters.

The compounds of the present invention, however, offer the possibility to affectively reduce catheter-related bacterial infections with a low risk of resistance development due to a novel therapeutic strategy targeting highly sensitive signal transduction mechanisms in bacteria. The preferred form of application is the coating and/or impregnating of catheter materials on both the inner and outer catheter surfaces. More preferably, the compounds of Formula (I) can be included in a mixture of antibacterial agents released continously from a catheter-associated depot into the environment. In a further embodiment, the compounds of the present invention and their pharmacologically acceptable salts can be administered directly to animals, preferably to mammals, and in particular to humans as antibiotics per se, as mixtures with one another or in the form of pharmaceutical preparations which allow enteral or parenteral use and which as active constituent contain an effective dose of at least one compound of the Formula (I) or (X), or a salt thereof, in addition to customary pharmaceutical excipients and additives. The compounds of Formula (I) or (X), can also be administered in form of their salts, which are obtainable by reacting the respective compounds with physiologically acceptable acids and bases.

The therapeutics can be administered orally, e.g., in the form of pills, tablets, coated tablets, sugar coated tablets, lozenges, hard and soft gelatin capsules, solutions, syrups, emulsions or suspensions or as aerosol mixtures. Administration, however, can also be carried out rectally, e.g., in the form of suppositories, or parenterally, e.g., in the form of injections or infusions, or percutaneously, e.g., in the form of ointments, creams or tinctures.

In addition to the active compounds of Formula (I) or (X), the pharmaceutical composition can contain further customary, usually inert carrier materials or excipients. Thus, the pharmaceutical preparations can also contain additives or adjuvants commonly used in galenic formulations, such as, e.g., filters, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for modifying the osmotic pressure, coating agents or antioxidants. They can also contain two or more compounds of the Formula (I) or their pharmacologically acceptable salts and also other therapeutically active substances.

Thus, the compounds of the present invention can be used alone, in combination with other compounds of this invention or in combination with other active compounds, for example with active ingredients already known for the treatment of the afore mentioned diseases, whereby in the latter case a favorable additive effect is noticed. Suitable amounts to be administered to mammalian in particular humans range from 5 to 1000 mg.

To prepare the pharmaceutical preparations, pharmaceutically inert inorganic or organic excipients can be used To prepare pills, tablets, coated tablets and hard gelatin capsules, e.g., lactose, corn starch or derivatives thereof, talc, stearic acid or its salts, etc. can be used Excipients for soft gelatin capsules and suppositories are, e.g., fats, waxes, semi-solid and liquid polyols, natural or hardened oils etc. Suitable excipients for the production of solutions and syrups are, e.g., water, alcohol, sucrose, invert sugar, glucose, polyols etc. Suitable excipients for the production of injection solutions are, e.g., water, alcohol, glycerol, polyols or vegetable oils.

The dose can vary within wide limits and is to be suited to the individual conditions in each individual case. For the above uses the appropriate dosage will vary depending on the mode of administration, the particular condition to be treated and the effect desired. In general, however, satisfactory results are achieved at dosage rates of about 0,1 to 100 mg/kg animal body weight preferably 1 to 50 mg/kg. Suitable dosage rates for larger mammal, e.g., humans, are of the order of from about 10 mg to 3 g/day, conveniently administered once, in divided doses 2 to 4 times a day, or in sustained release form.

In general, a daily dose of approximately 0,1 mg to 5000 mg, preferably 10 to 500 mg, per mammalian in particular human individual is appropriate in the case of the oral administration which is the preferred form of administration according to the invention. In the case of other administration forms too, the daily dose is in similar ranges. The compounds of Formula (I) can also be used in the form of a precursor (prodrug) or a suitably modified form, that releases the active compound in vivo.

In a further embodiment, the compounds of the present invention can be used as pharmacologically active components or ingredients of medical devices, instruments and articles with an effective dose of at least one compound of the Formula (I), or (X), or a salt thereof. The amount of the compounds used to coat for example medical device surfaces varies to some extent with the coating method and the application field. In general, however, the concentration range from about 0,01 mg per cm² to about 100 mg per cm². In a similar way the amount of the compounds has to be adjusted to the application mode if the compounds of the invention are used as components or ingredients in cleaning or treatment solutions. In general, effective dosages range from about 0,1 µM to about 1000 mM.

The following section shows examples for the synthesis of the compounds of the present invention and demonstrate their quorum sensing inhibiting effect.

### Examples

### 1. Synthesis of compounds of Formula (I) or (X),

### Synthesis method A (1,2-diacylhydrazine derivatives)

A solution of (1.2 eq) acid chloride or (1.2 eq) sulfonyl chloride in tetrahydrofuran was added to a solution of (1 eq) hydrazide in tetrahydrofuran and molecular sieve (0.4 nm) at 0°C. The mixture was stirred at room temperature. After 1 h the reaction mixture was concentrated in vacuum, and the resulting solid was purified by preparative thin layer chromatography (Merck, 20 x 20 cm, Silica gel 60 F₂₅₄, 1 mm) (CH₂Cl₂:MeOH, 100:1).

### Synthesis method B (1,2-diacylhydrazine derivatives)

A solution of (1.2 eq) acid chloride or (1.2 eq) sulfonyl chloride in dimethylformamide was added to a solution of (1 eq) hydrazide in dimethylformamide and (1.2 eq) triethylamine at 0°C. The mixture was stirred at room temperature. After 1 h the reaction mixture was concentrated in vacuum, and the resulting solid was purified by preparative thin layer chromatography (Merck, 20 x 20 cm, Silica gel 60 F₂₅₄, 1 mm) (CH₂Cl₂:MeOH, 100:1).

### Synthesis method C (1,2-diacylhydrazine derivatives)

A solution of (1.2 eq) acid chloride or (1.2 eq) sulfonyl chloride in dichloromethane was added to a solution of (1 eq) hydrazide in dichloromethane and (1.2 eq) triethylamine at 0°C. The mixture was stirred at room temperature. After 1 h the reaction mixture was concentrated in vacuum, and the resulting solid was purified by preparative thin layer chromatography (Merck, 20 x 20 cm, Silica gel 60 F₂₅₄, 1 mm) (*n*-hexano:EtOAc, 9:1).

### Synthesis method D (amide derivatives)

A solution of (1.2 eq) acid chloride in tetrahydrofuran was added to a solution of (1 eq) amine in tetrahydrofuran and molecular sieve (0.4 nm) at 0°C. The mixture was stirred at room temperature. After 1 h the reaction mixture was concentrated in vacuum, and the resulting solid was purified by preparative thin layer chromatography (Merck, 20 x 20 cm, Silica gel 60 F₂₅₄, 1 mm) (CH₂Cl₂:MeOH, 100:1).

### Synthesis method F (semicarbazide derivatives)

A solution of (1.3 eq) isocyanate or (1.3 eq) isothiocyanate in tetrahydrofuran was added to a solution of (1 eq) hydrazide in tetrahydrofuran and molecular sieve (0.4 nm) at 0°C The mixture was stirred at mom temperature. After 1 h the reaction mixture was concentrated in vacuum, and the resulting solid was purified by preparative thin layer chromatography (Merck, 20 x 20 cm, Silica gel 60 F₂₅₄,1 mm) (CH₂Cl₂:MeOH,100:5).

### Synthesis method G (hydrazide derivatives)

A solution of (1.2 eq) acid chloride in tetrahydrofuran was added to a solution of (1 eq) hydrazine in tetrahydrofuran and molecular sieve (0.4 nm) at 0°C. The mixture was stirred at room temperature. After 1 h the reaction mixture was concentrated in vacuum, and the resulting solid was purified by preparative thin layer chromatography (Merck, 20 x 20 cm, Silica gel 60 F₂₅₄, 1 mm) (CH₂Cl₂:MeOH,100:1).

In the following Table 2, the synthesis method employed in each case for the respective compound or whether the compound was obtained is indicated. Furthermore, the mass found by mass spectrometry, the exact molecular mass, the NMR-data (abbreviations: br. = broad, s = singulet, d = doublet, t = triplet, q = quartet, quint. = quintet, sext. = sextet, m_{c} = multiplet centered, m = multiplet, CHₐᵣ = aromatic H, *J*=¹H-¹H coupling constant) and the IC₅₀ range as a measure of anti-quorum sensing activity are indicated.

| | | | | | | |
|---|---|---|---|---|---|---|
| 27 | 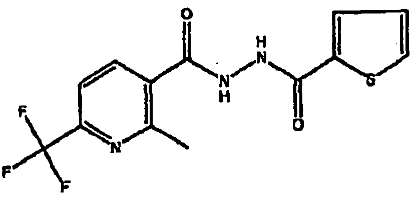 | Maybridge | 329 | - | - | +++ |
| 28 | 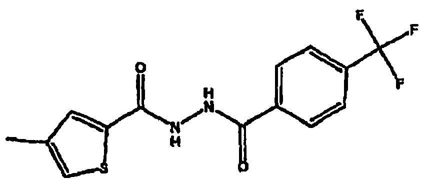 | Maybridge | 328 | - | - | ++ |
| 29 | 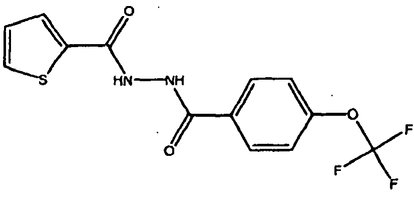 | A | 330 | 331 [M+H]⁺ | δ (DMSO-d₆) = 7.21 (t, *J*= 4.5 Hz, 1 H, H-4), 7.52 (d, *J*= 8.7 Hz, 2 H, CHₐᵣ), 7.85 (d, *J* = 4.8 Hz, 1 H, H-3), 7.88 (d, *J*= 3.6 Hz, 1 H, H-5), 8.03 (d, *J* = 8.7 Hz, 2 H, CHₐᵣ), 10.57 (s, 1 H, NH), 10.62 (s, 1 H, NH) | + |
| | | | | 329 [M-H]⁺ | | |
| 30 | 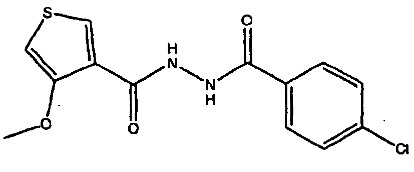 | Maybridge | 310 | - | - | + |
| 31 | 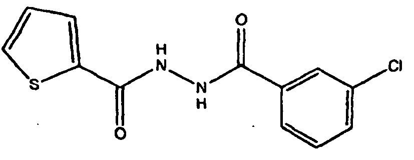 | Maybridge | 280 | - | - | ++ |
| 32 | 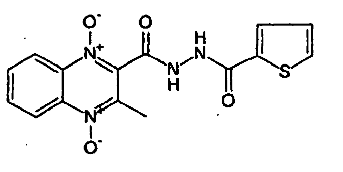 | Maybridge | 346 | - | - | ++ |
| 33 | 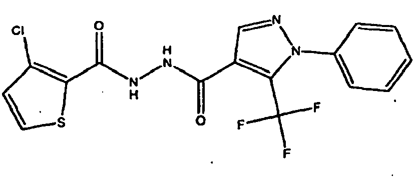 | B | 414 | 415 [M+H]⁺ | δ (CD₃OD) = 7.15 (d, *J*= 5.3 Hz, 1 H, H-4), 7.53-7.55 (m, 2 H, CHₐᵣ), 7.61-7.63 (m, 3 H, CHₐᵣ), 7.80 (d, *J* = 5.3 Hz, 1 H, H-5), 8.19 (s, 1 H, H-3') | + |
| | | | | 413 [M-H]⁺ | | |
| 34 | 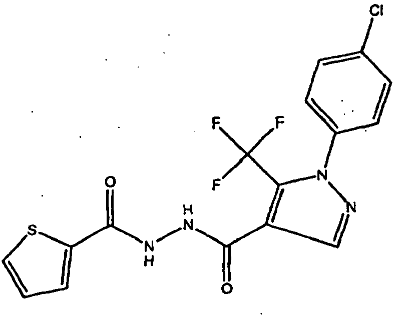 | A | 414 | 415 [M+H]⁺ | *δ* (CD₃OD) = 7.05 (t, *J*= 5.1 Hz, 1 H, H-4), 7.38 (d, *J*= 9.0 Hz, 2 H, CHₐᵣ), 7.47 (d, *J*= 9.0 Hz, 2 H, CHₐᵣ), 7.62 (dd, *J*= 5.1, 1.2 Hz,1 H, H-3), 7.70 (dd, *J*= 4.2, 1.5 Hz, 1 H, H-5), 8.05 (s, 1 H, H-3') | ++ |
| | | | | 413 [M-H]⁺ | | |
| 35 | 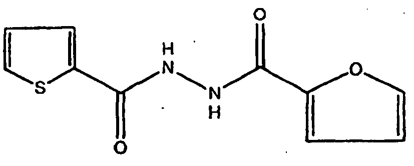 | TimTec | 236 | 237 [M+H]⁺ | δ (DMSO-d₆) = 3.45 (dd, *J*= 1.8, 3.3 Hz, 1 H, CHₐᵣ), 7.23 (dd, *J* = 3.6, 4.8 Hz, 1 H, CHₐᵣ), 7.29 (d, *J*= 3.6 Hz, 1 H, CHₐᵣ), 7.87-7.95 (m, 3 H, CHₐᵣ), 10.44 (s, 1 H, NH), 10.52 (s, 1 H, NH) | ++ |
| | | | | 235 [M-H]⁺ | | |
| 36 | 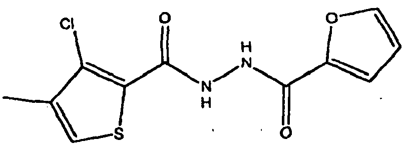 | Maybridge | 284 | - | - | ++ |
| 37 | 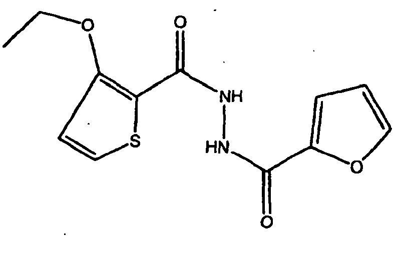 | Maybridge | 280 | - | - | ++ |
| 38 | 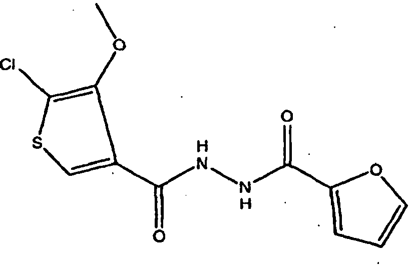 | A | 300 | 301 [M+H]⁺ | δ (DMSO-d₆) = 3.89 (s, 3 H, OCH₃), 6.67 (dd, *J*= 1.5, 3.3 Hz, 1 H, H-4), 7.26 (d, *J*= 3.6 Hz, 1 H, H-3), 7.24 (d, *J*= 3.3 Hz, 1 H, H-3), 7.91 (d, *J* = 1.5 Hz, 1 H, H-5), 7.94 (s, 1 H, H-3'), 10.12 (br. s, 1 H, NH), 10.36 (br. s, 1 H, NH) | +++ |
| | | | | 299 [M-H]⁺ | | |
| 39 | 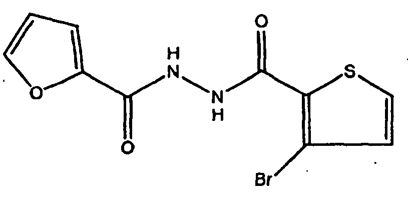 | A | 314 | 315 [M+H]⁺ | δ (DMSO-d₆) = 6.66 (dd, *J*= 1.8, 3.3 Hz, 1 H, H-4), 7.22 (d, *J*= 5.1 Hz, 1 H, H-4'), 7.24 (d, *J*= 3.3 Hz, 1 H, H-3), 7.58 (d, *J* = 5.1 Hz, 1 H, H-5'), 7.90 (d, *J* = 1.8 Hz, 1 H, H-5), 10.37 (br. s, 2 H, NH) | +++ |
| | | | | 313 [M-H]⁺ | | |
| 40 | 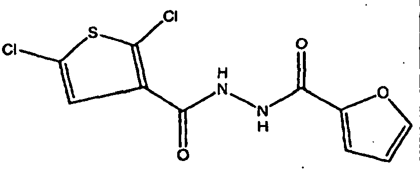 | A | 304 | 305 [M+H]⁺ | δ (DMSO-d₆) = 6.59 (dd, *J*= 1.8, 3.3 Hz, 1 H, H-4), 7.17 (d, *J*= 3.6 Hz, 1 H, H-3), 7.39 (s, 1 H, H-4'), 7.83 (d, *J*= 1.8 Hz, 1 H, H-5), 10.25 (s, 1 H, NH), 10.38 (s, 1 H, NH) | +++ |
| | | | | 303 [M-H]⁺ | | |
| 41 | 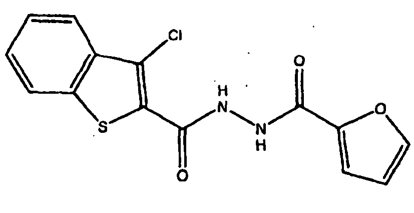 | TimTec | 320 | 321 [M+H]⁺ | δ (DMSO-d₆) = 6.52 (dd, *J*= 1.8, 3.6 Hz, 1 H, H-4), 7.13 (dd, *J*= 1.8, 3.6 Hz, 1 H, H-3), 7.44-7.47 (m, 2 H, CHₐᵣ), 7.76-7.79 (m, 2 H, CHₐᵣ), 7.99 (m_{c}, 1 H, H-5), 10.35 (s, 1 H, NH), 10.42 (s, 1 H, NH) | +++ |
| | | | | 319 [M-H]⁺ | | |
| 42 | 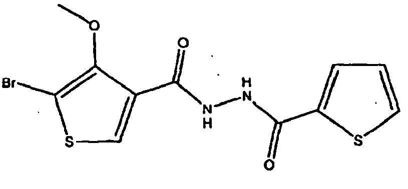 | Maybridge | 360 | - | - | +++ |
| 43 | 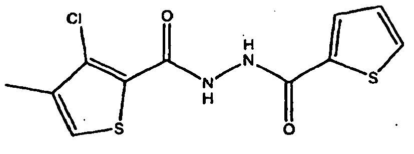 | Maybridge | 300 | - | - | +++ |
| 44 | 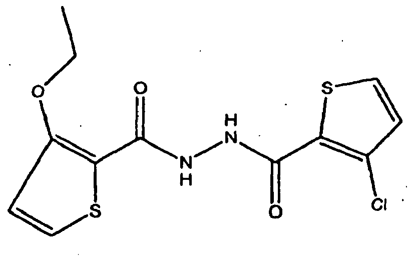 | B | 330 | 331 [M+H]⁺ | δ (CDCl₃) = 1.51 (t, *J*= 7.0 Hz, 3 H, OCH₂CH₃), 4.24 (q, *J* = 7.1 Hz, 2 H, O*CH₂*CH₃), 6.80 (d, *J*= 5.4 Hz, 1 H, H-4), 6.96 ( d, *J* = 5.3 Hz, 1 H, H-4'), 7.41 (d, *J* = 5.4 Hz, 1 H, H-5), 7.46 (d, *J* = 5.3 Hz, 1 H, H-5'), 9.97 (d, *J*= 7.3 Hz, 1 H, NH), 10.15 (d, *J* = 7.3 Hz, 1 H, NH) | + |
| | | | | 329 [M-H]⁺ | | |
| 45 | 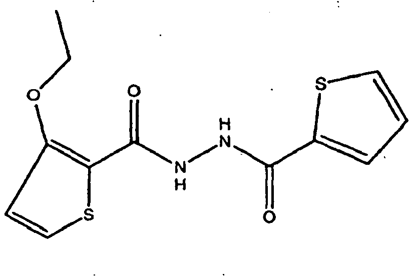 | B | 296 | 297 [M+H]⁺ | δ (CDCl₃) = 1.50 (t, *J*= 7.0 Hz, 3 H, OCH₂*CH₃*), 4.25 (q, *J*= 7.0 Hz, 2 H, OCH₂CH₃), 6.80 (d, *J*= 5.5 Hz, 1 H, H-4), 7.01 (dd, *J* = 6.0, 3.8 Hz, 1 H, H-4'), 7.40 (d, *J*= 5.4 Hz, 1 H, H-5), 7.43 (d, *J*= 3.9 Hz, 1 H, H-3'), 7.62 (d, *J*= 3.9 Hz, 1 H, H-5'), 9.50 (br. s, 1 H, NH), 9.90 (d, *J* = 5.3 Hz, 1 H, NH) | +++ |
| | | | | 295 [M-H]⁺ | | |
| 46 | 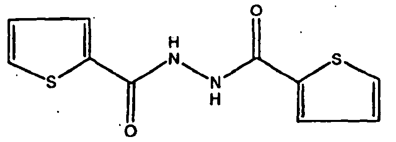 | AsInEx. | 252 | - | - | ++ |
| 47 | 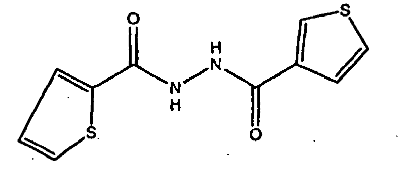 | A | 252 | 253 [M+H]⁺ | δ (CD₃OD) = 7.06 (t, *J* = 3.9 Hz, 1H, H-4), 7.41-7.48 (m, 2 H, H-4' and H-5'), 7.63 (d, *J*= 4.8 Hz, 1 H, H-3), 7.72 (d, *J*= 4.8 Hz, 1 H, H-5), 8.08 (s, 1 H, H-2') | +++ |
| | | | | 251 [M-H]⁺ | | |
| 48 | 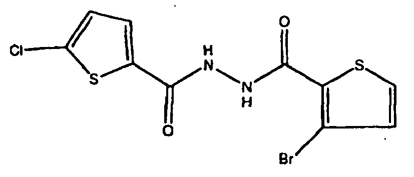 | A | 364 | 365 [M+H]⁺ | δ (CDCl₃) = 6.87 (d, *J*= 4.1 Hz, 1H, H-2'), 7.03 (d, *J* = 5.2 Hz, 1 H, H-1), 7.47 (d, *J*= 5.2 Hz, 1 H, H-2), 7.49 (d, *J*= 4.1 Hz, 1 H, H-1') | ++ |
| | | | | 363 [M-H]⁺ | | |
| 49 | 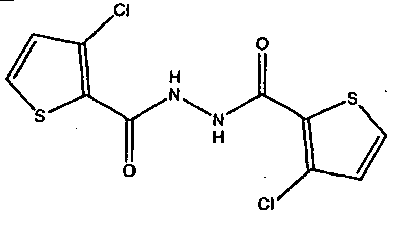 | A | 320 | 319 [M-H]⁺ | δ (CD₃OD) = 7.21 (d, *J*= 5.4 Hz, 2 H, H-4 and H-4'), 7.84 (d, *J*= 5.4 Hz, 2 H, H-5 and H-5') | ++ |
| 50 | 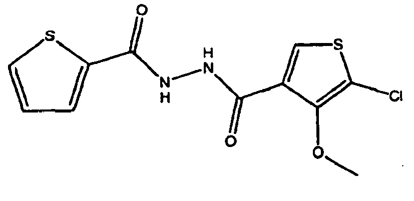 | B | 316 | 315 [M-H]⁺ | δ (CD₃OD) = 4.05 (s, 3 H, OCH₃), 7.16 (dd, *J*= 5.1, 3.9 Hz, 1 H, H-4'), 7.72 (dd, *J*= 5.1, 1.2 Hz, 1 H, H-3'), 7.81 (dd, *J*= 3.9, 1.2 Hz, 1 H, H-5'), 7.96 (s, 1 H, H-5) | ++ |
| 51 | 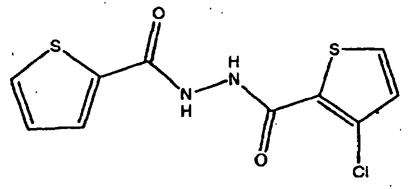 | SPECS and BioSPECS | 286 | - | - | ++ |
| 52 | 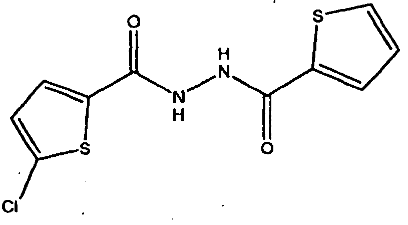 | A | 286 | 285 [M-H]⁺ | δ (DMSO-d₆) = 7.08 (d, *J*= 3.9 Hz, 1 H, H-4'), 7.13 (d, *J* = 4.2 Hz, 1 H, H-4), 7.68 (d, *J* = 3.9 Hz, 1 H, H-3), 7.74 (d, *J*= 3.9 Hz, 1 H, H-3'), 7.78 (d, *J*= 3.2 Hz, 1 H, H-5'), 10.55 (s, 2 H, NH) | ++ |
| 53 | 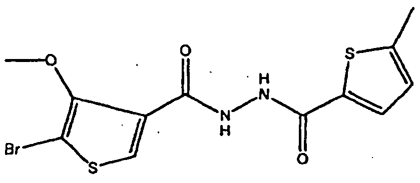 | A | 374 | 375 [M+H]⁺ | δ (CD₃OD)= 2.44 (s, 3 H, CH₃), 3.93 (s, 3 H, OCH₃), 6.75 (dd, *J*= 1.2, 3.9 Hz, 1 H, H-4), 7.52 (d, *J* = 3.6 Hz, 1 H, H-3), 8.01 (s, 1 H, H-5') | +++ |
| | | | | 373 [M-H]⁺ | | |
| 54 | 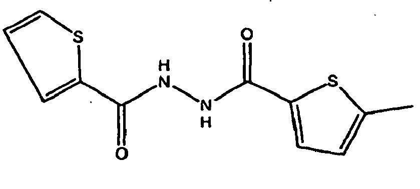 | A | 266 | 267 [M+H]⁺ | δ (CD₃OD) = 2,52 (s, 3 H, CH₃), 6.84 (d, *J* = 3.3 Hz, 1 H, H-4'), 7.16 (t, *J*= 4.8 Hz, 1 H, H-4), 7.61 (d, *J*= 3.6 Hz, 1 H, H-3'), 7.72 (d, *J* = 4.8 Hz, 1 H, H-3), 7.80 (d, *J*= 4.2 Hz, 1 H, H-5) | +++ |
| | | | | 265 [M-H]⁺ | | |
| 55 | 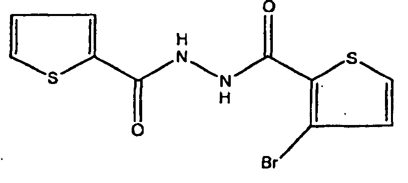 | A | 330 | 331 [M+H]⁺ | δ (CD₃OD) = 7.04-7.08 (m, 2 H, H-4 and H-4'), 7.58-7.65 (m, 2 H, H-5 and H-3'), 7.70 (d, *J* = 3.6 Hz, 1H, H-5') | +++ |
| | | | | 329 [M-H]⁺ | | |
| 56 | 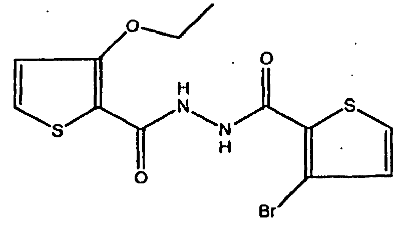 | B | 374 | 375 [M+H]⁺ | δ (CD₃OD) = 1.41 (t, *J*= 6.9 Hz, 3 H, CH₃), 4.27 (q, *J*= 6.9 Hz, 2 H, OCH₂), 6.98 (d, *J* = 5.4 Hz, 1 H, CHₐᵣ), 7.07 (d, *J*= 5.4 Hz, 1 H, CHₐᵣ), 7.59 (d, *J*= 5.7 Hz, 1 H, CHₐᵣ), 7.63 (d, *J* = 5.4 Hz, 1 H, CHₐᵣ) | +++ |
| | | | | 373 [M-H]⁺ | | |
| 57 | 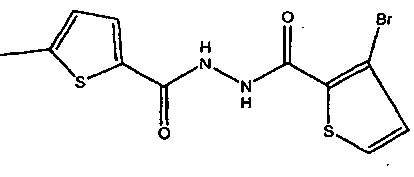 | A | 344 | 345 [M+H]⁺ | δ (CD₃OD) = 2.64 (s, 3 H, CH₃), 6.96 (dd, *J* = 0.9, 3.6 Hz, 1 H, CHₐᵣ), 7.26 (d, *J=* 5.1 Hz, 1 H, CHₐᵣ), 7.72 (d, *J* = 3.6 Hz, 1 H, CHₐᵣ), 7.82 (d, *J*= 5.1 Hz, 1 H, CHₐᵣ) | +++ |
| | | | | 343 [M-H]⁺ | | |
| 58 | 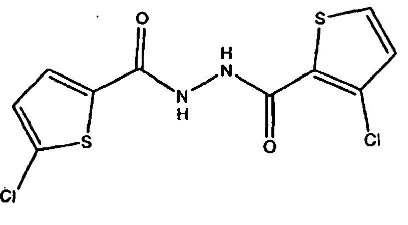 | A | 320 | 319 [M-H]⁺ | δ (DMSO-d₆) = 7.10 (d, *J* = 5.1 Hz, 1 H, H-4), 7.13 (d, *J* = 4.2 Hz, 1 H, H-4'), 7.60 (d, *J* = 3.9 Hz, 1 H, H-3'), 7.80 (d, *J* = 5.1 Hz, 1 H, H-5) | ++ |
| 59 | 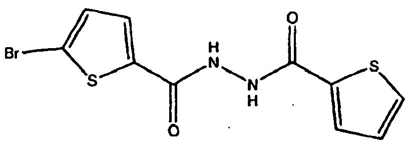 | TimTec | 330 | - | - | +++ |
| 60 | 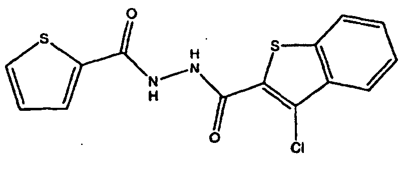 | TimTec | 336 | - | - | +++ |
| 61 | 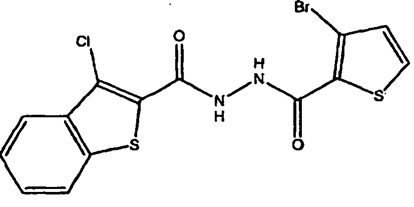 | B | 414 | 415 [M+H]⁺ | δ (CD₃OD) = 7.01 (d, *J* = 5.4 Hz, 1 H, H-4'), 7.39-7.43 (m, 2 H, CHₐᵣ), 7.56 (d, *J*=.5.1 Hz, 1 H, H-5'), 7.78-7.82 (m, 2 H, CHₐᵣ) | ++ |
| | | | | 413 [M-H]⁺ | | |
| 62 | 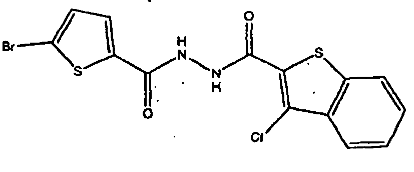 | B | 414 | 413 [M-H]⁺ | δ (CDCl₃) = 7.03 (d, *J*= 3.7 Hz, 1 H, H-4), 7.40 (d, *J*= 3.2 Hz, 1H, H-3), 7.45-7.48 (m, 2 H, CHₐᵣ), 7.77-7.87 (m, 2 H, CHₐᵣ), 9.14 (s, 1 H, NH), 9.79 (s, 1 H, NH) | ++ |
| 63 | 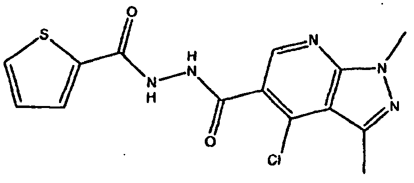 | B | 349 | 350 [M+H]⁺ | δ (CD₃OD) = 2.73 (s, 3 H, CH₃), 4.05 (s, 3 H, NCH₃), 7.18 (dd, *J* = 5.1, 3.6 Hz, 1 H, H-4'), 7.75 (dd, *J*= 5.1, 1.2 Hz, 1 H, H-3'), 7.83 (dd, *J* = 3.6, 1.2 Hz, 1 H, H-5'), 8.68 (s, 1 H, H-6) | + |
| | | | | 348 [M-H]⁺ | | |
| 64 | 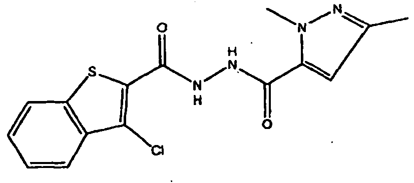 | B | 348 | 347 [M-H]⁺ | δ (CD₃OD) = 2.28 (s, 3 H, CH₃), 4.08 (s, 3 H, NCH₃), 6.70 (s, 1 H, H-4), 7.57-7.62 (m, 2 H, CHₐᵣ), 7.96-8.01 (m, 2 H, CHₐᵣ ) | + |
| 65 | 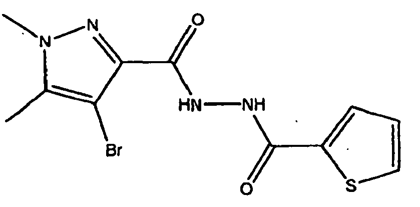 | Maybridge | 342 | - | - | +++ |
| 66 | 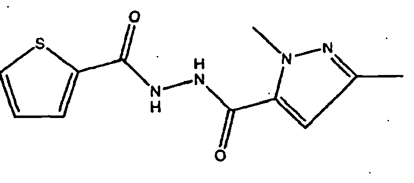 | B | 264 | 265 [M+H]⁺ | δ (CD₃OD) = 2.25 (s, 3 H, CH₃), 4.04 (s, 3 H, NCH₃), 6.65 (s, 1 H, H-4), 7.17 (dd, *J*= 5.1, 3.9 Hz, 1 H, H-4'), 7.73 (dd, *J* = 5.1,0.9 Hz, 1 H, H-3'), 7.79 (d, *J*= 3.9 Hz, 1 H, H-5') | + |
| | | | | 263 [M-H]⁺ | | |
| 67 | 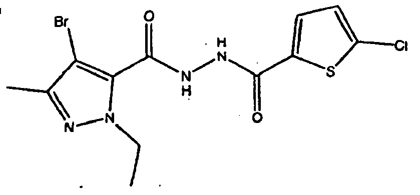 | A | 390 | 391 [M+H]⁺ | δ (CDCl₃) = 1.33 (t, *J*= 7.1 Hz, 3 H, NCH₂*CH₃*), 1.83 (s, 3 H, CH₃), 4.42 (dd, *J*= 14.3, 7.2 Hz, 2 H, N*CH*₂CH₃), 6.85 (d, *J* = 4.1 Hz, 1 H, H-4), 7.44 (d, *J* = 4.1 Hz, 1 H, H-3) | + |
| | | | | 389 [M-H]⁺ | | |
| 68 | 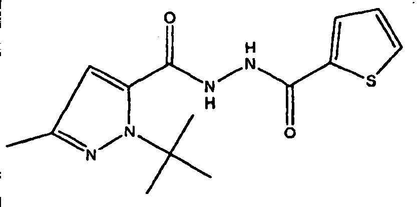 | A | 306 | 305 [M-H]⁺ | δ (CD₃OD) = 1.68 (s, 9 H, -C(CH₃)₃), 2.86 (s, 3 H, CH₃), 6.47 (s, 1 H, H-4'), 7.17 (t, *J*= 3.9 Hz, 1 H, H-4), 7.74 (dd, *J* = 4.2, 0.9 Hz, 1 H, H-3), 7.82 (dd, *J*= 3.6, 0.9 Hz, 1 H, H-5) | + |
| 69 | 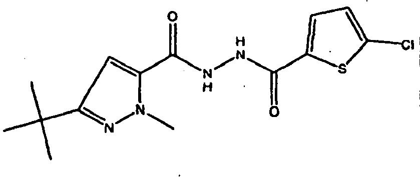 | B | 340 | 341 [M+H]⁺ | δ (CDCl₃) = 1.22 (s, 9 H, tBu), 4.00 (s, 3 H, NCH₃), 6.58 (s, 1 H, H-4'), 6.81 (d, *J*= 3.9 Hz, 1 H, H-4), 7.44 (d, *J* = 3.9 Hz, 1 H, H-3) | +++ |
| | | | | 339 [M-H]⁺ | | |
| 70 | 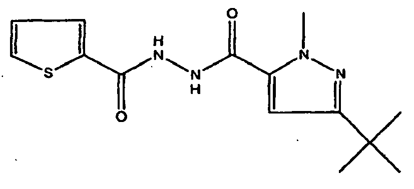 | A | 306 | 307 [M+H]⁺ | δ (CD₃OD) = 1.33 (s, 9 H, -C(CH₃)₃), 4.08 (s, 3 H, NCH₃), 6.80 (s, 1 H, H-4'), 7.18 (t, *J*= 3.6 Hz, 1 H, H-4), 7.75 (dd, *J* = 5.1,1.2 Hz, 1 H, H-3), 7.82 (d, *J*= 3.6 Hz, 1 H, H-5) | + |
| | | | | 305 [M-H]⁺ | | |
| 71 | 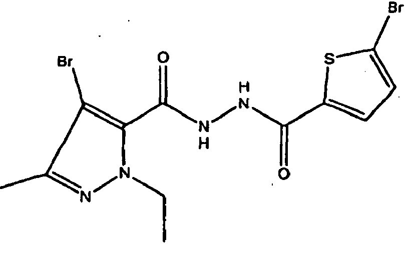 | A | 434 | 435 [M+H]⁺ | δ (CD₃OD) = 1.31 (t, *J*= 7.5 Hz, 3 H, NCH₂*CH₃*), 2.13 (s, 3 H, CH₃), 4.23 (q, *J*= 7.2 Hz, 2 H, N*CH₂*CH₃), 7.10 (d, *J*= 4.2 Hz, 1 H, H-4), 7.48 (d, *J* = 3.9 Hz, 1 H, H-3) | +++ |
| | | | | 433 [M-H]⁺ | | |
| 72 | 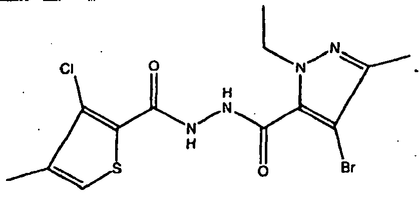 | A | 404 | 405 [M+H]⁺ | δ (CD₃OD) = 1.29 (t, *J*= 6.9 Hz, 3 H, NCH₂*CH₃*), 2.12 (s, 3 H, CH₃), 2.13 (s, 3 H, CH₃), 4.23 (q, *J* = 7.2 Hz, 2H, N*CH₂*CH₃), 7.36 (s, 1 H, H-5) | + |
| | | | | 403 [M-H]⁺ | | |
| 73 | 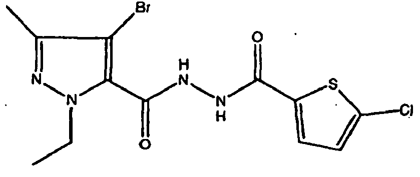 | A | 390 | 391 [M+H]⁺ | δ (CD₃OD) = 1.28 (t, *J* = 7.2 Hz, 3 H, NCH₂C*H*₃), 2.10 (s, 3 H, CH₃), 4.20 (q, *J* = 6.9 Hz, 2 H, NCH₂C*H*₃), 6.94 (d, *J* = 4.2 Hz, 1 H, H-4), 7.50 (d, *J*= 4.2 Hz, 1 H, H-3) | +++ |
| | | | | 389 [M-H]⁺ | | |
| 74 | 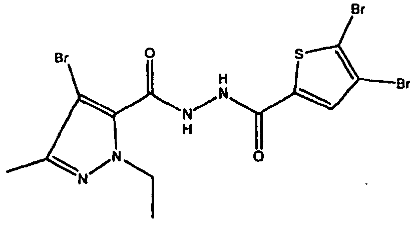 | A | 512 | 513 [M+H]⁺ | δ (DMSO-d₆)= 1.37 (t, *J* = 7.2 Hz, 3 H, NCH₂C*H*₃), 2.23 (s, 3 H, CH₃), 4.28 (q, *J*= 7.2 Hz, 2 H, NC*H*₂CH₃), 7.94 (s, 1 H, H-3), 10.75 (s, 1 H, NH), 11.09 (s, 1 H, NH) | + |
| | | | | 511 [M-H]⁺ | | |
| 75 | 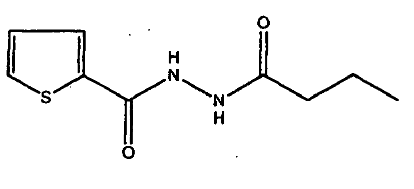 | TimTec | 212 | 213 [M+H]⁺ | δ (CD₃OD) = 0.90 (t, *J*= 7.5 Hz, 3 H, CH₃), 1.60 (sext., *J* = 7.5 Hz, 2 H, CH₂), 2.18 (t, *J* = 7.2 Hz, 2 H, CH₂), 7.04 (dd, *J* = 3.9, 5.1 Hz, 1 H, H-4), 7.60 (dd, *J*= 3.9, 5.1 Hz, 1 H, H-3), 7.65 (dd, *J*= 3.9, 5.1 Hz, 1 H, H-5) | +++ |
| | | | | 211 [M-H]⁺ | | |
| 76 | 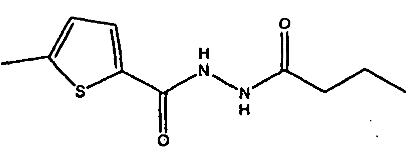 | A | 226 | 227 [M+H]⁺ | δ (CD₃OD) = 1.01 (t, *J* = 7.5 Hz, 3 H, CH₃), 1.71 (sext., *J*= 7.5 Hz, 2 H, CH₂), 2.28 (t, *J* = 7.5 Hz, 2 H, CH₂), 2.52 (s, 3 H, CH₃), 6.82 (d, *J*= 3.9 Hz, 1 H, H-4), 7.57 (d, *J*= 3.6 Hz, 1 H, H-3) | ++ |
| | | | | 225 [M-H]⁺ | | |
| 77 | 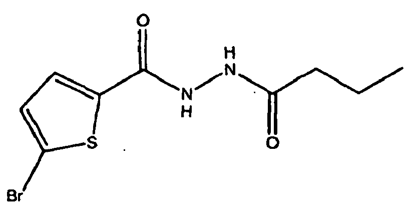 | A | 290 | 291 [M+H]⁺ | δ (CD₃OD) = 0.90 (t, *J*= 7.5 Hz; 3 H, CH₃), 1.59 (sext., *J*= 7.8 Hz, 2 H, CH₂), 2.17 (t, *J* = 7.5 Hz, 2 H, CH₂), 7.05 (d, *J*= 3.9 Hz, 1 H, H-4), 7.43 (d, *J*= 4.2 Hz, 1 H, H-3) | +++ |
| | | | | 289 [M-H]⁺ | | |
| 78 | 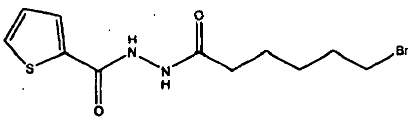 | A | 318 | 319 [M+H]⁺ | δ (DMSO-d₆) = 1.41 (m_{c}, 2 H, H-3'), 1.56 (m_{c}, 2 H, H-2'), 1. 81 (m_{c}, 2 H, H-4'), 2.17 (t, *J* = 7.2 Hz, 2 H, H-1'), 3.52 (t, *J*= 6.6 Hz, 2 H, H-5'), 7.16 (t, *J*= 4.5 Hz, 1 H, H-4), 7.81 (d, *J* = 4.2 Hz, 2 H, H-3 and H-5), 9.82 (s, 1 H, NH), 10.28 (s, 1 H, NH) | +++ |
| 79 | 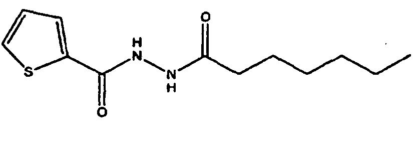 | A | 254 | 255 [M+H]⁺ | δ (DMSO-d₆) = 0.86 (t, *J*= 6.9 Hz, 2 H, H-6'), 1.20-1.36 (m, 6 H, H-3', H-4' and H-5'), 1.53 (m_{c}, 2 H, H-2'), 2.17 (t, *J*= 7.2 Hz, 2H, H-1'), 7.16 (t, *J*= 4.5 Hz, 1 H, H-4), 7.80-7.84 (m, 2 H, H-3 and H-5), 9.79 (s, 1 H, NH), 10.27 (s, 1 H, NH) | +++ |
| | | | | 253 [M-H]⁺ | | |
| 80 | 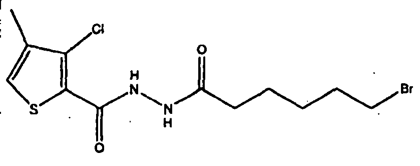 | C | 366 | 367 [M+H]⁺ | δ (CDCl₃) = 1.45 (m*_{c}*, 1 H, H-3'), 1.68 (m*_{c}*, 1 H, H-2'), 1.80 (m*_{c}*, 1 H, H-4'), 2.17 ( s, 3 H, CH₃), 2.35 (t, *J*= 7.4 Hz, 1 H, H-1'), 3.33 (t, *J* = 6.7 Hz, 1H, H-5'), 7.19 (s, 1 H, H-5), 9.66 (d, *J* = 6.4 Hz, 1 H, NH), 9.82 (d, *J* = 6.3 Hz, 1 H, NH) | +++ |
| | | | | 365 [M-H]⁺ | | |
| 81 | 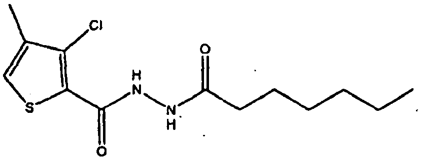 | C | 302 | 303 [M+H]⁺ | δ (CDCl₃) = 0.80 (t, *J*= 6.7 Hz, 1 H, H-6'), 1.18-1.28 (m, 3 H, H-3', H-4' and H-5'), 1.62 (m*_{c}*, 1 H, H-2'), 2.16 ( s, 3 H, CH₃), 2.31 (t, *J*=7.4 Hz, 1 H, H-1'), 7.19 (s, 1 H, H-5), 9.60 (d, *J* = 6.5 Hz, 1 H, NH), 9.84 (d, *J =* 6.4 Hz, 1 H, NH) | +++ |
| | | | | 301 [M-H]⁺ | | |
| 82 | 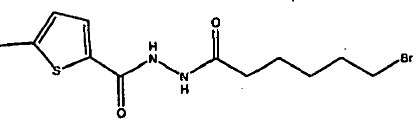 | A | 332 | 333 [M+H]⁺ | δ (DMSO-d₆) = 1.41 (m_{c}, 2 H, CH₂), 1.53 (m_{c}, 2 H, CH₂), 1.79 (quint., *J*= 7.2 Hz, 2 H, CH₂), 2.16 (t, *J* = 7.2 Hz, 2 H, CH₂), 2.46 (s, 3 H, CH₃), 3.52 (t, *J* = 6.9 Hz, 2 H, CH₂), 6.85 (dd, *J* = 0.9, 4.8 Hz, 1 H, H-4), 7.61 (d, *J* = 3.9 Hz, 1 H, H-3), 9.77 (s, 1 H, NH), 10.16 (s, 1 H, NH) | +++ |
| 83 | 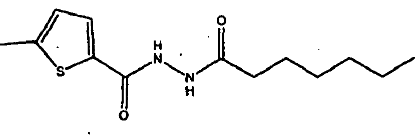 | A | 268 | 269 [M+H]⁺ | δ (DMSO-d₆) = 0.84 (t, *J*= 4.2 Hz, 3 H, CH₃), 1.24 (m_{c}, 6 H, (CH₂)₃), 1.48 (quint., *J*= 7.2 Hz, 2 H, CH₂), 2.18 (t, *J* = 7.5 Hz, 2 H, CH₂), 2.46 (s, 3 H, CH₃), 6.85 (d, *J*= 3.9 Hz, 1 H, H-4), 7.60 (d, *J* = 3.3 Hz, 1 H, H-3), 9.74 (s, 1 H, NH), 10.14 (s, 1 H, NH) | +++ |
| | | | | 267 [M-H]⁺ | | |
| 84 | 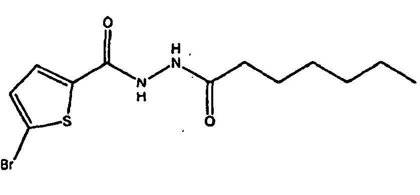 | A | 332 | 333 [M+H]⁺ | δ (CD₃OD) = 0.82 (t, *J*= 6.9 Hz, 3 H, CH₃), 1.19-1.34 (m, 6 H, (CH₂)₃), 1.56 (quint., *J* = 7.2 Hz, 2 H, CH₂), 2.19 (t, *J* = 7.5 Hz, 2 H, CH₂), 7.05 (d, *J* = 4.2 Hz, 1 H, H-4), 7.43 (d, *J*= 4.2 Hz, 1 H, H-3) | +++ |
| | | | | 331 [M-H]⁺ | | |
| 85 | 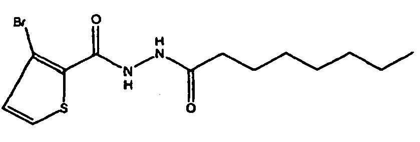 | A | 346 | 347 [M+H]⁺ | δ (DMSO-d₆) = 0.89 (t, *J*= 6.9 Hz, 3 H, CH₃), 1.30-1.37 (m, 8 H, (CH₂)₄), 1.57 (quint., *J*= 6.6 Hz, 2 H, CH₂), 2.19 (t, *J*= 7.5 Hz, 2 H, CH₂), 7.24 (d, *J*= 5.1 Hz, 1 H, H-4), 7.87 (d, *J* = 5.1 Hz, 1 H, H-5), 9.95-10.16 (br. s, 2 H, NH) | +++ |
| | | | | 345 [M-H]⁺ | | |
| 86 | 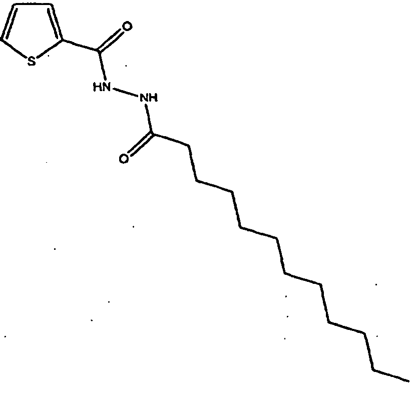 | A | 324 | 325 [M+H]⁺ | δ (CD₃OD) = 0.80 (t, *J*= 6.9 Hz, 3 H, CH₃), 1.14-1.32 (m, 16 H, (CH₂)₈), 1.57 (quint., *J* = 7.5 Hz, 2 H, CH₂), 2.20 (t, *J*= 7.5 Hz, 2 H, CH₂), 7.04 (dd, *J*= 3.9, 4.8 Hz, 1 H, H-4), 7.60 (dd, *J*= 0.9, 4.8 Hz, 1 H, H-3), 7.65 (dd, *J*= 1.2,4.2 Hz, 1 H, H-5) | +++ |
| | | | | 323 [M-H]⁺ | | |
| 87 | 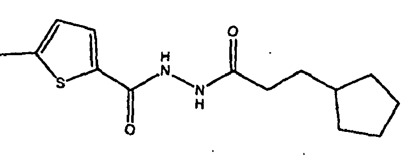 | A | 280 | 281 [M+H]⁺ | δ (DMSO-d₆) = 1.06 (m_{c}, 2 H, CH₂), 1.50 (m_{c}, 6 H, (CH₂)₃), 1.72 (m_{c}, 3 H, CH and CH₂), 2.16 (t, *J*= 7.8 Hz, 2 H, CH₂), 2.46 (s, 3 H, CH₃), 6.65 (dd, *J*= 0.9,3.9 Hz, 1 H, H-4), 7.61 (d, *J*= 3.6 Hz, 1 H, H-3), 9.75 (s, 1 H, NH), 10.14 (s, 1 H, NH) | +++ |
| | | | | 279 [M-H]⁺ | | |
| 88 | 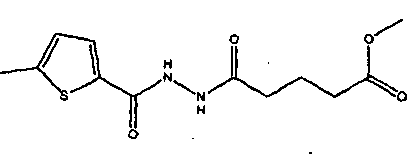 | A | 284 | 285 [M+H]⁺ | δ (CD₃OD) = 1.96 (t, *J* = 7.2 Hz, 2 H, CH₂), 2.36 (t, *J*= 7.5 Hz, 2 H, CH₂), 2.44 (t, *J* = 7.5 Hz, 2 H, CH₂), 2.51 (s, 3 H, CH₃), 3.67 (s, 3 H, OCH₃), 6.82 (d, *J*= 3.9 Hz, 1 H, H-4), 7.57 (d, *J*= 3.6 Hz, 1 H, H-3) | + |
| | | | | 283 [M-H]⁺ | | |
| 89 | 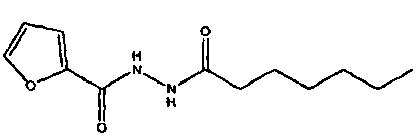 | A | 238 | 239 [M+H]⁺ | δ (CD₃OD) = 1.11 (t, *J*= 6.6 Hz, 3 H, CH₃), 1.47-1.63 (m, 6 H, (CH₂)₃), 1.86 (quint., *J* = 7.2 Hz, 2 H, CH₂), 2.48 (t, *J* = 7.5 Hz, 2 H, CH₂), 6.79 (br. s, 1 H, H-4), 7.39 (d, *J*= 3.6 Hz, 1 H, H-3), 7.88 (br. s, 1 H, H-5) | +++ |
| | | | | 237 [M-H]⁺ | | |
| 90 | 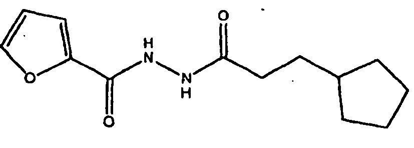 | A | 250 | 251 [M+H]⁺ | δ (DMSO-d₆) = 1.14 (m_{c}, 2 H, CH₂), 1.60 (m_{c}, 6 H, (CH₂)₃), 1.80 (m_{c}, 3 H, CH and CH₂), 2.23 (t, *J* = 7.2 Hz, 2 H, CH₂), 6.70 (dd, *J* = 1.8, 3.6 Hz, 1 H, H-4), 7.62 (d, *J* = 3.6 Hz, 1 H, H-3), 7.94 (d, *J* = 2.7 Hz, 1 H, H-5), 9.82 (s, 1 H, NH), 10.19 (s, 1 H, NH) | +++ |
| | | | | 249 [M-H]⁺ | | |
| 91 | 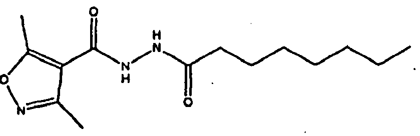 | A | 281 | 282 [M+H]⁺ | δ (DMSO-d₆) = 0.75 (t, *J* = 6.9 Hz, 3 H, CH₃), 1.15-1.23 (m, 8 H, (CH₂)₄), 1.43 (quint., *J*= 6.9 Hz, 2 H, CH₂), 2.05 (t, *J*= 7.8 Hz, 2 H, CH₂), 2.17 (s, 3 H, CH₃), 2.42 (s, 3 H, CH₃), 9.71 (s, 1 H, NH), 9.76 (s, 1 H, NH) | +++ |
| | | | | 280 [M-H]⁺ | | |
| 92 | 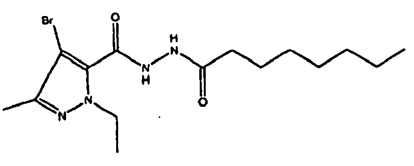 | A | 372 | 373 [M+H]⁺ | δ (DMSO-d₆) = 0.65 (t, *J*= 6.6 Hz, 3 H, CH₃), 0.97-1.12 (m, 11 H, NCH₂CH₃ and (CH₂)₄), 1.33 (quint., *J* = 6.9 Hz, 2 H, CH₂), 1.92-1.97 (m, 5 H, CH₃-3 and CH₂) 3.98 (q, *J*= 7.5 Hz, 2 H, NCH₂), 9.79 (s, 1 H, NH), 10.09 (s, 1 H, NH) | ++ |
| | | | | 371 [M-H]⁺ | | |
| 93 | 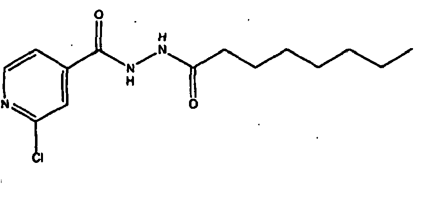 | A | 297 | 298 [M+H]⁺ | δ (DMSO-d₆) = 0.81 (t*, J* = 7.2 Hz, 3 H, CH₃), 1.16-1.27 (m, 8 H, (CH₂)₄), 1.48 (m_{c}, 2 H, CH₂), 2.12 (t, *J* = 7.5 Hz, 2 H, CH₂), 7.71 (dd, *J*= 1.5, 5.1 Hz, 1 H, H-5), 7.80 (br. s, 1 H, H-3), 8.54 (d, *J* = 5.1 Hz, 1 H, H-6), 9.94 (s, 1 H, NH), 10.62 (s, 1 H, NH) | +++ |
| | | | | 296 [M-H]⁺ | | |
| 94 | 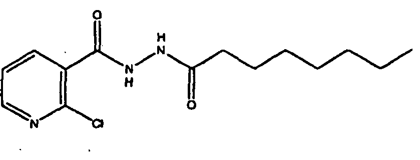 | A | 297 | 298 [M+H]⁺ | δ (DMSO-d₆) = 0.94 (t, *J*= 7.2 Hz, 3 H, CH₃), 1.34-1.39 (m, 8 H, (CH₂)₄), 1.62 (quint., *J*= 6.9 Hz, 2 H, CH₂), 2.25 (t, *J* = 7.2 Hz, 2 H, CH₂), 7.61 (dd, *J* = 5.1, 7.8 Hz, 1H, H-5), 7.98 (dd, *J*= 1.8, 7.5 Hz, 1 H, H-4), 8.59 (dd, *J*= 1.8,4.5 Hz, 1 H, H-6), 10.12 (s, 1 H, NH), 10.47 (s, 1 H, NH) | +++ |
| | | | | 296 [M-H]⁺ | | |
| 95 | 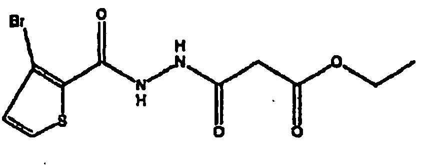 | A | 334 | 335 [M+H]⁺ | δ (DMSO-d₆) = 0.96 (t, *J* = 7.2 Hz, 3 H, CH₃), 3.09 (s, 2H, CH₂), 3.86 (q, *J*= 7.2 Hz, 2 H, OCH₂), 6.97 (d, *J=* 5.4 Hz, 1 H, H-4), 7.60 (d, *J*= 5.1 Hz, 1 H, H-5), 10.05 (s, 2H, NH) | +++ |
| | | | | 333 [M-H]⁺ | | |
| 96 | 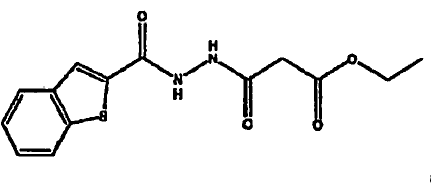 | A | 306 | 307 [M+H]⁺ | δ (DMSO-d₆) =1.33 (t, *J*= 7.2 Hz, 3 H, CH₃), 3.49 (s, 2 H, CH₂), 4.24 (q, *J*= 7.2 Hz, 2 H, OCH₂), 7.55-7.64 (m, 2 H, CHₐᵣ), 8.10 (d, *J*= 6.8 Hz, 1 H, CHₐᵣ), 8.17 (d, *J* = 6.9 Hz, 1 H, CHₐᵣ), 8.30 (s, 1 H, H-3), 10.39 (s, 1 H, NH), 10.92 (s, 1 H, NH) | ++ |
| | | | | 305 [M-H]⁺ | | |

### 2. Biosensor Assay

Quorum sensing inhibition of the compounds was investigated with the aid of the bioluminescent sensor strain *Escherichia coli* MT102 (pSB403) (Winson et al., FEMS Microbiol. Lett. 163:185-92, 1998). Plasmid pSB403 contains the *Photobacterium fischeri luxR* gene together with the *luxI* promoter region as a transcriptional fusion to the bioluminescence genes *luxCDABE* of *Photorhabdus luminescence.* Although *E. coli* pSB403 exhibits the highest sensitivity for the *Photobacterium fischeri* quorum sensing signal *N*-(3-oxohexanoyl) homoserine lactone (3-oxo-C6-HSL), a wide range of other HSL molecules are detected by the sensor (Winson et al., FEMS Microbiol. Lett. 163:185-92, 1998; Geisenberger et al., FEMS Microbiol. Lett. 184:273-8, 2000).

Inhibitory studies were conducted in a microtitre dish assay as follows: the *E. coli* sensor strain grown over night in LB medium (Sambrook et al., Molecular Cloning: A Laboratory Maual. 2nd Edn. Cold Spring Harbor Laboratory, New York, 1989) was diluted 1:4 and grown for another 1 hour at 30°C. After addition of 3-oxo-C6-HSL (final concentration 100 nM) 100 µl of an exponential culture suspension were filled in the wells of a FluoroNunc Polysorp microtitre dish. The test compounds were added to the culture in different concentrations and bioluminescence was measured after 4 hours of incubation at 30°C with a Lamda Fluoro 320 Plus reader (Bio-Tek Instruments). Inhibitor-mediated reduction of light emission was correlated with the value obtained without addition of the test compounds. IC₅₀ values (concentration of inhibitor required for 50% inhibition of the signal compared to the signal without inhibitor) were determined by using a fitting function after drawing a graph of the activities of eight different inhibitor concentrations. The determined IC₅₀ range of each compound is listed in Table 2.

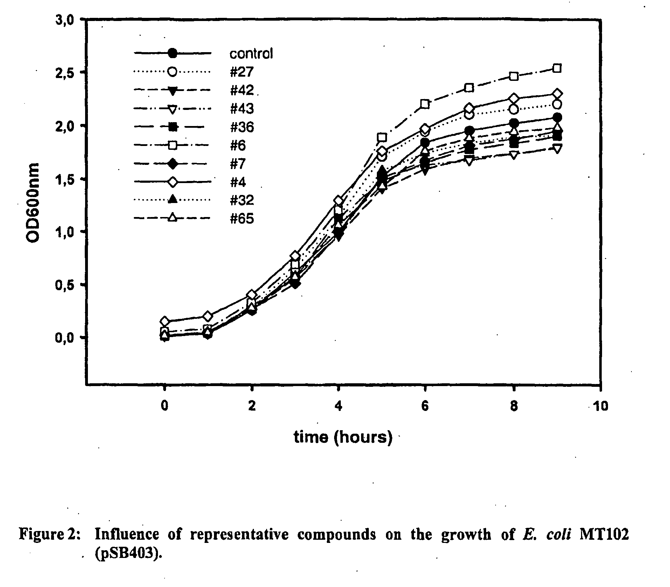

To exclude the possibility that the inhibitory effect is attributed to growth inhibition but not to a specific interaction of the test compound with the sensors quorum sensing system growth curves in the presence and absence of the test compounds were compared. *E. coli* MT102 (pSB403) was grown in LB medium at 37°C in the presence of 0,4 mM test compound. Growth was measured as optical density at 600 nm. None of the compounds listed in Table 2 exhibit any growth inhibitory effects on the sensor strain *E. coli* MT102 (pSB403). Figure 2 shows the growth curves of representative compounds indicating a specific inhibitory effect of the compounds on the quorum sensing system.

### 3. Inhibition of protease production

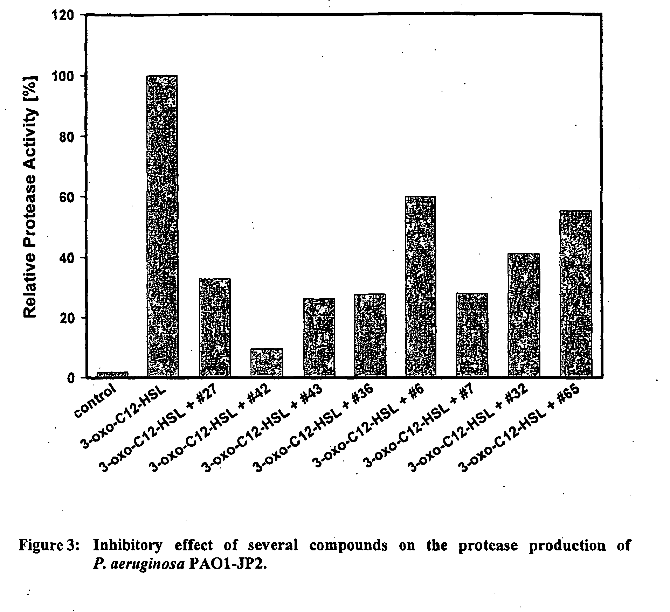

The inhibitory effect of the compounds on quorum sensing regulated virulence factors was demonstrated by investigating the expression of extracellular proteases by *Pseudomonas aeruginosa.* The *P. aeruginosa* mutant strain PAO-JP2 (Pearson et al., J. Bacteriol. 179:5756-67, 1997) carrying mutations in the quorum sensing genes *lasI* and *rhlI* is unable to produce extracellular proteolytic enzymes. Protease expression can be completely restored by external addition of 3-oxo-C12-HSL. The protease assay was performed according to Riedel et al. (J. Bacteriol. 183:1805-9, 2001) with few modifications. PAO-JP2 was grown in LB medium at 30°C and shaking at 250 rpm to an OD₆₀₀ₙₘ of 0,5. The test compounds were added at a final concentration of 0,4 mM and the culture was incubated for further 30 min at 30°C and shaking at 250 rpm. After addition of 3-oxo-C12-HSL at a final concentration of 0,3 µM the cultures were grown for an additional 6 hours at 30°C and shaking at 250 rpm. The proteolytic activity was measured as described by Ayora & Götz (Mol. Gen. Genet. 242:421-30, 1994). 50 µl culture supernatant were incubated with Azocasein (250 µl 2%, Sigma, St. Louis, Mo.) for 1 hour at 37°C. After precipitation of undigested substrate with trichloroacetic acid (1,2 ml 10%) for 20 minutes at room temperature, followed by 5 minutes centrifugation at 13000 rpm, NaOH (0,75 ml 1M) was added to the supernatant. The relative protease activity was measured as absorbance at 440 nm (OD₄₄₀ₙₘ) of the supernatant divided by the optical density of the culture (OD₆₀₀ₙₘ). Figure 3 demonstrates the inhibitory effect of several compounds on protease production of *P. aeruginosa* PAO-JP2. The data presented are representative for at least three separate experiments.

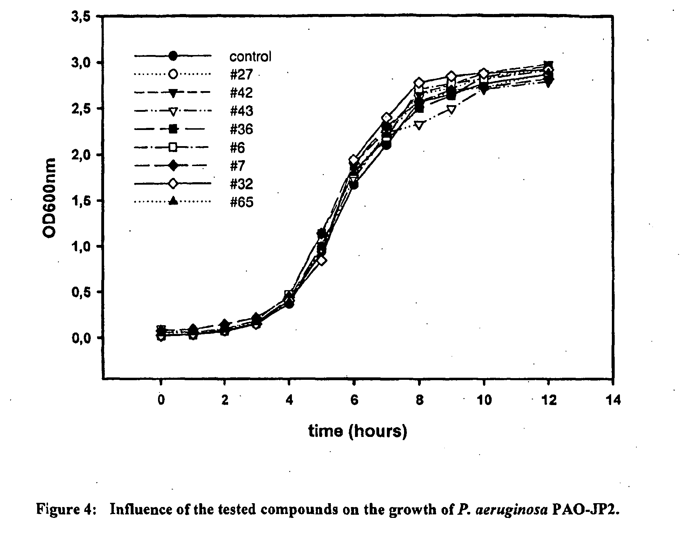

To demonstrate that inhibition of protease production is due to a specific interference with the quorum sensing system growth curves in the presence and absence of the test compounds were compared. *P. aeruginosa* PAO-JP2 was grown in LB medium at 30°C in the presence of 0,4 mM test compound. Growth was measured as optical density at 600 nm. None of the compounds listed in Table 2 exhibit any growth inhibitory effects on *P. aeruginosa* PAO-JP2. Figure 4 shows the growth curves of representative compounds indicating a specific inhibitory effect of the compounds on the quorum sensing system.

### 4. Inhibition of biofilm formation

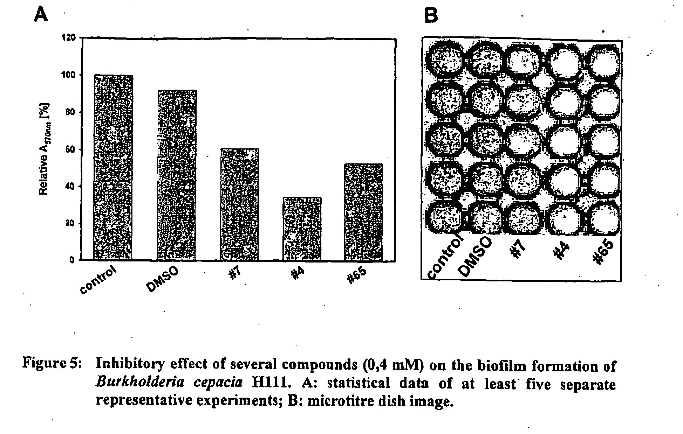

The bacterial biofilm formation assay was performed in polystyrene microtitre dishes (FluoroNunc Polysorp) according to the method described by OToole & Kolter (Mol. Microbiol. 28:449-61, 1998) and Pratt & Kolter (Mol. Microbiol. 30:285-93, 1998) with few modifications (Huber et al., Microbiology, 147:2517-28, 2001). Cells were grown in the wells of the microtitre dishes in 100 µl AB medium (Clark & Maaloe, J. Mol. Biol. 23:99-112, 1967) supplemented with 10 mM sodium citrate (Sigma). After addition of the test compound (0,4 mM) the cells were incubated for 48 hours at 30°C. The medium was then removed and 100 µl of a 1% (w/v) aqueous solution of crystal violet (Merck) was added. Following staining at room temperature for 20 minutes, the dye was removed and the wells were washed thoroughly with water. For quantification of attached cells, the crystal violet was solubilized in a 80:20 (v/v) mixture of ethanol and acetone and the absorbance was determined at 570 nm (Ultrospec Plus spectrometer, Pharmacia). Figures 5A and 5B demonstrate the inhibitory effect of several compounds on biofilm formation of *Burkholderia cepacia* H111 (Römling et al., J. Infect. Dis. 170:1616-21, 1994; Gotschlich et al., Syst. Appl. Microbiol. 24:1-14, 2001). The data presented are representative for at least five separate experiments.

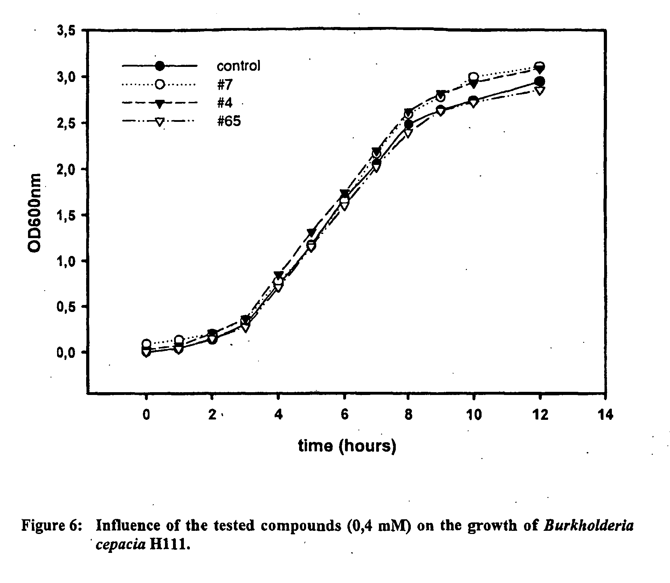

To exclude the possibility that biofilm inhibition is attributed to growth inhibition growth curves in the presence and absence of the test compounds were compared. *Burkholderia cepacia* H111 was grown in LB medium at 37°C in the presence of 0,4 mM test compound. Growth was measured as optical density at 600 nm. None of the compounds listed in Table 2 exhibit any growth inhibitory effects on the sensor strain *Burkholderia cepacia* H111. Figure 6 shows the growth curves of the tested compounds indicating a specific inhibitory effect of the compounds on the quorum sensing system.

## Claims

1. The use of compounds of the general Formula (I) 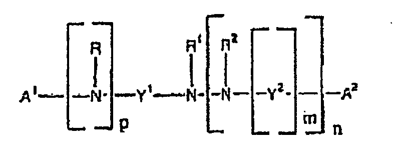 for regulation of the quorum sensing system of microorganisms wherein in Formula (I),
R¹ is H;
R² is H;
A¹ and A² each independently represent an C₁-C₂₀-alkyl, C₁-C₂₀-alkenyl , or C₁-C₂₀-alkynyl group which may contain one or more group(s) Z, or a monocyclic or polycyclic aromatic or non-aromatic ring system which may contain one or more group(s) X, and in case of a polycyclic ring system, said system contains at least one aromatic ring;
Z is selected from the group consisting of S, 0, N, NR⁴, CO, CO₂, CS, SO or SO₂;
X is selected from the group consisting of S, 0, N, NR⁴, SO or SO₂;
said ring system may carry a substituent R³ on one or more of the carbon atoms of said ring system;
said C₁-C₂₀-alkyl, C₁-C₂₀-alkenyl, or C₁-C₂₀-alkynyl group may carry a substituent R³ on one or more of the carbon atoms of said group;
R³ is independently H, OR⁴, SR⁴, hydroxyalkyl, hydroxyalkylamine, cycloalkyl, halogen, haloalkyl, haloalkyloxy, NO₂, CN, SO₂NR⁴R⁵, CO₂NR⁴R⁵, COR⁴, CO₂R⁴, SO₂R⁴, SO₃R⁴, NR⁴R⁵, alkyl , alkenyl, alkynyl, aryl or heteroaryl;
R⁴ is H, alkyl, alkenyl, alkynyl, cycloalkyl, aryl or heteroaryl;
R⁵ is H, O-alkyl, O-aryl, alkyl, alkenyl, alkynyl, heteroaryl or aryl;
Y¹ and Y² are C=O;
p is 0, m is 1, n is 1;
wherein
an alkyl group denotes a linear or branched C₁-C₆-alkyl group which may be substituted by one or more substituents R³;
an alkenyl group denotes a linear or branched C₁-C₆-alkenyl group which may be substituted by one or more substituents R³;
an alkynyl group denotes a linear or branched C₁-C₆-alkynyl group which may be substituted by one or more substituents R³;
a cycloalkyl group denotes a non-aromatic ring system containing three to eight carbon atoms, wherein one or more of the carbon atoms in the ring may be substituted by a group X, X being as defined above;
an alkoxy group denotes an O-alkyl group, the alkyl group being as defined above;
a haloalkyl group denotes an alkyl group which is substituted by one to five halogen atoms, the alkyl group being as defined above;
a hydroxyalkyl group denotes an HO-alkyl group, the alkyl group being as defined above;
a haloalkyloxy group denotes an alkoxy group which is substituted by one to five halogen atoms, the alkoxy group being as defined above;
a hydroxyalkylamino group denotes an (HO-alkyl)₂-N group or HO-alkyl-NH group, the alkyl group being as defined above;
an aryl group denotes an aromatic group having five to fifteen carbon atoms which may be substituted by one or more substituents R³, R³ being as defined above;
a heteroaryl group denotes a 5- or 6-membered heterocyclic group which contains at least one heteroatom selected from O, N, and S, wherein the heterocyclic group may be fused to another ring; with the proviso that the treatment of the human or animal body is excluded.

2. The use according to claim 1 for the treatment of biofilms or for inhibiting biofilm formation.

3. The use according to claim 1 or 2 for the treatment of biofilms or for inhibiting biofilm formation on medical articles, instruments and devices.

4. The use according to claim 1 or 2 for the treatment of biofilms or for inhibiting biofilm formation in disinfectants, cleaning and treatment solutions.

5. The use according to claim 1 or 2 for the treatment of biofilms or for inhibiting biofilm formation in personal hygiene articles, toiletries and cosmetics.

6. The use according to claim 1 or 2 for the treatment of biofilms or for inhibiting biofilm formation in industrial settings.

7. The use according to claim 1 or 2 for the treatment of biofilms or for inhibiting biofilm formation in industrial settings wherein the industrial setting is selected from the group consisting of ship hulls, food processing systems, oil recovery or paper manufacturing plants.

8. The use according to claim 1 or 2 for the treatment of, biofilms or for inhibiting biofilm formation in environmental settings.

9. The use according to claim 1 or 2 for the treatment of biofilms or for inhibiting biofilm formation in environmental settings wherein the environmental setting is selected from the group consisting of water distribution or cooling water systems.

10. The use of compounds of the general Formula (I) 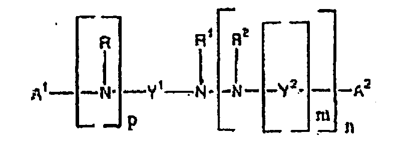 for the preparation of a medicament, antibacterial agent or antifouling coating for the treatment or prevention of bacterial damages and diseases,
wherein in Formula (I),
R¹ is H;
R² is H;
A¹ and A² each independently represent an C₁-C₂₀-alkyl, C₁-C₂₀-alkenyl, or C₁-C₂₀-alkynyl group which may contain one or more group(s) Z, or a monocyclic or polycyclic aromatic or non-aromatic ring system which may contain one or more group(s) X, and in case of a polycyclic ring system, said system contains at least one aromatic ring;
Z is selected from the group consisting of S, O, N, NR⁴, CO, CO₂, CS, SO or SO₂;
X is selected from the group consisting of S, O, N, NR⁴, SO or SO₂ ;
said ring system may carry a substituent R³ on one or more of the carbon atoms of said ring system;
said C₁-C₂₀-alkyl, C₁-C₂₀-alkenyl, or C₁-C₂₀-alkynyl group may carry a substituent R³ on one or more of the carbon atoms of said group;
R³ is independently H, OR⁴, SR⁴, hydroxyalkyl, hydroxyalkylamine, cycloalkyl, halogen, haloalkyl, haloalkyloxy, NO₂, CN, SO₂NR⁴R⁵, CO₂NR⁴R⁵, COR⁴, CO₂R⁴, SO₂R⁴, SO₃R⁴, NR⁴R⁵, alkyl , alkenyl, alkynyl, aryl or heteroaryl;
R⁴ is H, alkyl, alkenyl, alkynyl, cycloalkyl, aryl or heteroaryl;
R⁵ is H, 0-alkyl, O-aryl, alkyl, alkenyl, alkynyl, heteroaryl or aryl;
Y¹ and Y² are C=O;
p is 0, m is 1, n is 1;
wherein
an alkyl group denotes a linear or branched C₁-C₆-alkyl group which may be substituted by one or more substituents R³;
an alkenyl group denotes a linear or branched C₁-C₆-alkenyl group which may be substituted by one or more substituents R³;
an alkynyl group denotes a linear or branched C₁-C₆-alkynyl group which may be substituted by one or more substituents R³;
a cycloalkyl group denotes a non-aromatic ring system containing three to eight carbon atoms, wherein one or more of the carbon atoms in the ring may be substituted by a group X, X being as defined above;
an alkoxy group denotes an 0-alkyl group, the alkyl group being as defined above;
a haloalkyl group denotes an alkyl group which is substituted by one to five halogen atoms, the alkyl group being as defined above;
a hydroxyalkyl group denotes an HO-alkyl group, the alkyl group being as defined above;
a haloalkyloxy group denotes an alkoxy group which is substituted by one to five halogen atoms, the alkoxy group being as defined above;
a hydroxyalkylamino group denotes an (HO-alkyl)₂-N group or HO-alkyl-NH group, the alkyl group being as defined above;
an aryl group denotes an aromatic group having five to fifteen carbon atoms which may be substituted by one or more substituents R³, R³ being as defined above;
a heteroaryl group denotes a 5- or 6-membered heterocyclic group which contains at least one heteroatom selected from 0, N, and S, wherein the heterocyclic group may be fused to another ring;
with the proviso that the treatment of the human or animal body is excluded.

11. The use according to claim 10, wherein the medicament, antibacterial agent or antifouling coating is for the treatment or prevention of bacterial damages and diseases caused by Gram-negative bacteria.

12. The use according to claim 10, wherein the medicament, antibacterial agent or antifouling coating is for the treatment or prevention of bacterial damages and diseases caused by *Pseudomonas aeruginosa* or *Burkholderia cepacia.*

13. A compound of the general Formula (X) and pharmaceutically acceptable salts thereof: 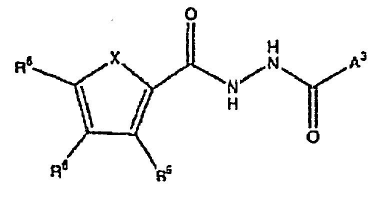 wherein
A³ is an C₈-C₂₀-alkyl, C₈-C₂₀-alkenyl, or C₈-C₂₀-alkynyl group or 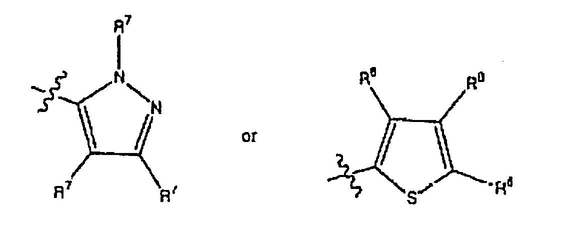 R⁶ is independently of each other -H, -F, -Cl, -Br, -I, -NO₂, - NR⁴R⁵, -CN, alkyl, cycloalkyl, -OH, alkoxy, alkylthio, hydroxyalkylamino, haloalkyl, haloalkyloxy, hydroxyalkyl, aryl, alkenyl, alkynyl, or heteroaryl;
R⁷ is independently of each other -H, -F, -Cl, -Br, -I, -NO₂,-NR⁴R⁵, -CN, alkyl , alkenyl, alkynyl, cycloalkyl, -OH, alkoxy, alkylthio, hydroxyalkylamino, haloalkyl, haloalkyloxy, hydroxyalkyl, aryl or heteroaryl;
R⁸ is independently of each other -H, -F, -Cl, -Br, -I, -NO₂,-NR⁴R⁵, -CN, alkyl, alkenyl, alkynyl, cycloalkyl, -OH, alkoxy, alkylthio, hydroxyalkylamino, haloalkyl, haloalkyloxy, hydroxyalkyl, aryl or heteroaryl;
X is selected from the group consisting of S, O, N, NR⁴, SO or SO₂;
R⁴ is H, alkyl, alkenyl, alkynyl, cycloalkyl, aryl or heteroaryl;
R⁵ is H, O-alkyl, 0-aryl, alkyl, alkenyl, alkynyl, heteroaryl or aryl;
wherein
an alkyl group denotes a linear or branched C₁-C₆-alkyl group which may be substituted by one or more substituents R³ ;
an alkenyl group denotes a linear or branched C₁-C₆-alkenyl group which may be substituted by one or more substituents R³;
an alkynyl group denotes a linear or branched C₁-C₆-alkynyl group which may be substituted by one or more substituents R³;
a cycloalkyl group denotes a non-aromatic ring system containing three to eight carbon atoms, wherein one or more of the carbon atoms in the ring may be substituted by a group X, X being as defined above;
an alkoxy group denotes an O-alkyl group, the alkyl group being as defined above;
a haloalkyl group denotes an alkyl group which is substituted by one to five halogen atoms, the alkyl group being as defined above;
a hydroxyalkyl group denotes an HO-alkyl group, the alkyl group being as defined above;
a haloalkyloxy group denotes an alkoxy group which is substituted by one to five halogen atoms, the alkoxy group being as defined above;
a hydroxyalkylamino group denotes an (HO-alkyl)₂-N group or HO-alkyl-NH group, the alkyl group being as defined above;
an aryl group denotes an aromatic group having five to fifteen carbon atoms which may be substituted by one or more substituents R³, R³ being as defined above;
a heteroaryl group denotes a 5- or 6-membered heterocyclic group which contains at least one heteroatom selected from 0, N, and S, wherein the heterocyclic group may be fused to another ring;
with the proviso that 2-thiophenecarboxylic acid-5-nitro-2-(2-thienylcarbonyl)hydrazide, 4-butylthiophene-2-carboxylic acid N'-(4-butyl-thiophen-2-carbonyl)hydrazide, 2-thiophenecarboxylic acid-3-chloro-2-(2-thienylcarbonyl)hydrazide, 2-thiophenecarboxylic acid-5-bromo-2-(2-thienylcarbonyl)hydrazide, 1H-pyrazole-5-carboxylic acid-1-methyl-2-(2-thienylcarbonyl)hydrazide, 2-thiophenedicarboxylic acid-5-(4,5,6,7-tetrahydrobenzo[b]thien-2-yl)-2-[[5-(4,5,6,7-tetrahydrobenzo[b]thien-2-yl)-2-thienyl]-carbonyl]hydrazide, 1H-pyrrole-2-carboxylic acid-2-(2-thienylcarbonyl)hydrazide, 2-thiophenecarboxylic acid-2-(2-thienylcarbonyl)hydrazide, 2-thiophene-carboxylic acid-N'-(furan-2-carbonyl)hydrazide, thiophene-2-carboxylic acid-N'-(3-chloro-4-methylthiophene-2-carbonyl)hydrazide, 2-furancarboxylic acid-5-[[[4-methyl-6-(trifluoromethyl)-2-pyrimidinyl] thio] methyl]-2-(2-thienylcarbonyl)hydrazide, 1H-pyrazole-3-carboxylic acid-4-bromo-1,5-dimethyl-2-(2-thienylcarbonyl)hydrazide and 2-thiophenecarboxylic acid-N'-(5-bromofuran-2-carbonyl)hydrazide are excluded.

14. A compound according to claim 13, including the compounds excluded by any of the disclaimers, for the use as a medicament, antibacterial agent or antifouling agent.

15. The use of a compound according to claim 13, including the compounds excluded by any of the disclaimers, for the preparation of a medicament, antibacterial agent or antifouling coating for the treatment or prevention of bacterial damages and diseases.

16. The use according to claim 15, wherein the medicament, antibacterial agent or antifouling coating is for the treatment or prevention of bacterial damages and diseases caused by Gram-negative bacteria.

17. The use according to claim 15, wherein the medicament, antibacterial agent or antifouling coating is for the treatment or prevention of bacterial damages and diseases caused by Pseudomonas aeruginosa or Burkholderia cepacia.

18. The use of a compound of claim 13 for the treatment of biofilms or for inhibiting biofilm formation.

19. The use according to claim 18 for the treatment of biofilms or for inhibiting biofilm formation on medical articles, instruments and devices.

20. The use according to claim 18 for the treatment of biofilms or for inhibiting biofilm formation in disinfectants, cleaning and treatment solutions.

21. The use according to claim 18 for the treatment of biofilms or for inhibiting biofilm formation in personal hygiene articles, toileteries and cosmetics.

22. The use according to claim 18 for the treatment of biofilms or for inhibiting biofilm formation in industrial settings.

23. The use according to claim 18 for the treatment of biofilms or for inhibiting biofilm formation in industrial settings wherein the industrial setting is selected from the group consisting of ship hulls, food processing systems, oil recovery or paper manufacturing plants.

24. The use according to claim 18 for the treatment of biofilms or for inhibiting biofilm formation in environmental settings.

25. The use according to claim 18 for the treatment of biofilms or for inhibiting biofilm formation in environmental settings wherein the environmental setting is selected from the group consisting of water distribution or cooling water systems.

## Patentansprüche

1. Verwendung von Verbindungen der allgemeinen Formel (I) 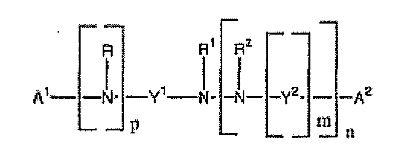 zur Regulierung des Quorum Sensing Systems von Mikroorganismen, wobei in Formel (I)
R¹ Wasserstoff ist;
R² Wasserstoff ist;
A¹ und A² jeweils unabhängig einen C₁-C₂₀-Alkyl-, C₁-C₂₀-Alkenyl- oder C₄-C₂₀-Alkinyl-Rest, welcher eine oder mehrere Gruppe(n) Z enthalten kann, oder ein monozyklisches oder polyzyklisches aromatisches oder nicht-aromatisches Ringsystem, welches eine oder mehrere Gruppe(n) X enthalten kann, darstellt, und wobei im Fall eines polyzyklischen Ringsystems dieses System mindestens einen aromatischen Ring enthält;
Z ausgewählt ist aus S, O, N, NR⁴, CO, CO₂, CS, SO oder SO_{2,}
X ausgewählt ist aus S, O, N, NR⁴, SO oder SO₂;
das Ringsystem einen Substituenten R³ an einem oder mehreren der Kolilenstoffatozne des Ringsystems tragen kann;
der C₁-C₂₀-Alkyl-, C₁-C₂₀-Alkenyl- oder C₁-C₂₀-Alkinyl-Rest einen Substituenten R³ an einem oder mehreren der Kohlenstoffatome dieses Rests tragen kann;
R³ unabhängig Wasserstoff, OR⁴, SR⁴, Hydroxyalkyl, Hydroxyalkylamin, Cycloalkyl, Halogen, Haloalkyl, Haloalkyloxy, NO₂, CN, SO₂NR⁴R⁵, CO₂NR⁴R⁵, COR⁴, CO₂R⁴, SO₂R⁴, SO₃R⁴, NR⁴R⁵, Alkyl, Alkenyl, Alkinyl, Aryl oder Heteroaryl ist;
R⁴ Wasserstoff, Alkyl, Alkenyl, Alkinyl, Cycloalkyl, Aryl oder Heteroaryl ist;
R⁵ Wasserstoff, O-Alkyl, O-Aryl, Alkyl, Alkenyl, Alkinyl, Heteroaryl oder Aryl ist;
Y¹ und Y² C=O sind;
p den Wert 0 hat, m den Wert 1 hat, n den Wert 1 hat;
wobei
ein Alkylrest einen linearen oder verzweigten C₁-C₆-Alkylrest bezeichnet, welcher mit einem oder mehreren Substituenten R³ substituiert sein kann;
ein Alkenylrest einen linearen oder verzweigten C₁-C₆-Alkenylrest bezeichnet, welcher mit einem oder mehreren Substituenten R³ substituiert sein kann;
ein Alkinylrest einen linearen oder verzweigten C₁-C₆-Alkinylrest bezeichnet, welcher mit einem oder mehreren Substituenten R³ substituiert sein kann;
ein Cycloalkylrest ein drei bis acht Kohlenstoffatome enthaltendes nichtaromatisches Ringsystem bezeichnet, wobei eines oder mehrere der Kohlenstoffatome im Ring durch eine Gruppe X substituiert sein können, wobei X wie vorstehend definiert ist;
ein Alkoxyrest einen O-Alkylrest darstellt, wobei der Alkylrest wie vorstehend definiert ist;
ein Haloalkylrest einen mit einem bis fünf Halogenatomen substituierten Alkylrest bezeichnet, wobei der Alkylrest wie vorstehend definiert ist;
ein Hydroxyalkylrest einen HO-Alkylrest bezeichnet, wobei der Alkylrest wie vorstehend definiert ist;
ein Haloalkyloxyrest einen mit einem bis rund Halogenatomen substituierten Alkoxyrest bezeichnet, wobei der Alkoxyrest wie vorstehend definiert ist;
ein Hydroxyalkylaminorest einen (HO-Alkyl)₂N-Rest oder HO-Alkyl-NH-Rest bezeichnet, wobei der Alkylrest wie vorstehend definiert ist;
ein Arylrest einen aromastischen Rest mit fünf bis fünfzehn Kohlenstoffatomen darstellt, welcher mit einem oder mehreren Substituenten R³ substituiert sein kann, wobei R³ wie vorstehend definiert ist;
ein Heteroarylrest einen 5- oder 6-gliedrigen heterocyclischen Rest darstellt, welcher mindestens ein aus O, N und S ausgewählte Heteroatom enthält, wobei der heterocyclische Rest an einen weiteren Ring anelliert sein kann;
mit der Maßgabe, dass die Behandlung des menschlichen oder tierischen Körpers ausgeschlossen ist.

2. Verwendung gemäß Anspruch 1 zur Behandlung von Biofilmen oder zur Inhibierung von Biofilm-Bildung.

3. Verwendung gemäß Anspruch 1 oder 2 zur Behandlung von Biofilmen oder zur Inhibierung von Biofilm-Bildung auf medizinischen Gegenständen, Instrumenten und Geräten.

4. Verwendung gemäß Anspruch 1 oder 2 zur Behandlung von Biofilmen oder zur Inhibierung von Biofilm-Bildung in Desinfektionsmitteln, Reinigungs- und Behandlungslösungen.

5. Verwendung gemäß Anspruch 1 oder 2 zur Behandlung von Biofilmen oder zur Inhibierung von Biofihn-Bildung bei Gegenständen für die Körperpflege, Toilettenartikeln und Kosmetika.

6. Verwendung gemäß Anspruch 1 oder 2 zur Behandlung von Biofilmen oder zur Inhibierung von Biofilm-Bildung im industriellen Bereich.

7. Verwendung gemäß Anspruch 1 oder 2 zur Behandlung von Biofilmen oder zur Inhibierung von Biofilm-Bildung im industriellen Bereich, wobei der industrielle Bereich ausgewählt ist aus Schiffskörpern, Nahrungsmittel- Verarbeitungssystemen, Ölgewinnungs- oder Papierherstellungsanlagen.

8. Verwendung gemäß Anspruch 1 oder 2 zur Behandlung von Biofilmen oder zur Inhibierung von Biofilm-Bildung im Umweltbereich.

9. Verwendung gemäß Anspruch 1 oder 2 zur Behandlung von Biofilmen oder zur Inhibierung von Biofilm-Bildung im Umweltbereich, wobei der Umweltbereich ausgewählt ist aus Wasserverteilungs- oder Kühlwassersystemen.

10. Verwendung von Verbindungen der allgemeinen Formel (I) 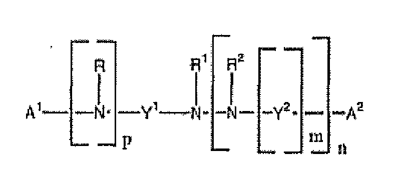 zur Herstellung eines Medikaments, antibakteriellen Mittels oder einer Antifouling-Beschichtung zur Behandlung oder Vorbeugung von bakteriellen Schäden und Erkrankungen,
wobei in Formel (I)
R¹ Wasserstoff ist;
R² Wasserstoff ist;
A¹ und A² jeweils unabhängig einen C₁-C₂₀-Alkyl-, C₁-C₂₀-Alkenyl- oder C₁-C₂₀-Alkinyl-Rest, welcher eine oder mehrere Gruppe(n) Z enthalten kann, oder ein monozyklisches oder polyzyklisches aromatisches oder nicht-aromatisches Ringsystem, welches eine oder mehrere Gruppe(n) X enthalten kann, darstellt, und wobei im Fall eines polyzyklischen Ringsystems dieses System mindestens einen aromatischen Ring enthält;
Z ausgewählt ist aus S, O, N, NR⁴, CO, CO₂, CS, SO oder SO₂,
X ausgewählt ist aus S, O, N, NR⁴, SO oder SO₂;
das Ringsystem einen Substituenten R³ an einem oder mehreren der Kohlenstoffatotne des Ringsystems tragen kann;
der C₁-C₂₀-Alkyl-, C₁-C₂₀-Alkenyl- oder C₁-C₂₀-Alkinyl-Rest einen Substituenten R³ an einem oder mehreren der Kohlenstoffatome dieses Rests tragen kann;
R³ unabhängig Wasserstoff, OR⁴, SR⁴, Hydroxyalkyl, Hydroxyalkylamin, Cycloalkyl, Halogen, Haloalkyl, Haloalkyloxy, NO₂, CN, SO₂NR⁴R⁵, CO₂NR⁴R⁵, COR⁴, CO₂R⁴, SO₂R⁴, SO₃R⁴, NR⁴R⁵, Alkyl, Alkenyl, Alkinyl, Aryl oder Heteroaryl ist;
R⁴ Wasserstoff, Alkyl, Alkenyl, Alkinyl, Cycloalkyl, Aryl oder Heteroaryl ist;
R⁵ Wasserstoff, O-Alkyl, O-Aryl, Alkyl, Alkenyl, Alkinyl, Heteroaryl oder Aryl ist;
Y¹ und Y² C=O sind;
p den Wert 0 hat, m den Wert 1 hat, n den Wert 1 hat;
wobei
ein Alkylrest einen linearen oder verzweigten C₁-C₆-Alkylrest bezeichnet, welcher mit einem oder mehreren Substituenten R³ substituiert sein kann;
ein Alkenylrest einen linearen oder verzweigten C₁-C₆-Alkenylrest bezeichnet, welcher mit einem oder mehreren Substituenten R³ substituiert sein kann;
ein Alkinylrest einen linearen oder verzweigten C₁-C₆-Alkinylrest bezeichnet, welcher mit einem oder mehreren Substituenten R³ substituiert sein kann;
ein Cycloalkylrest ein drei bis acht Kohlenstoffatome enthaltendes nichtaromatisches Ringsystem bezeichnet, wobei eines oder mehrere der Kohlenstoffatome im Ring durch eine Gruppe X substituiert sein können, wobei X wie vorstehend definiert ist;
ein Alkoxyrest einen O-Alkylrest darstellt, wobei der Alkylrest wie vorstehend definiert ist;
ein Haloalkylrest einen mit einem bis fünf Halogenatomen substituierten Alkylrest bezeichnet, wobei der Alkylrest wie vorstehend definiert ist;
ein Hydroxyalkylrest einen HO-Alkylrest bezeichnet, wobei der Alkylrest wie vorstehend definiert ist;
ein Haloalkyloxyrest einen mit einem bis fünf Halogenatomen substituierten Alkoxyrest bezeichnet, wobei der Alkoxyrest wie vorstehend definiert ist;
ein Hydroxyalkylaminorest einen (HO-Alkyl)₂N-Rest oder HO-Alkyl-NH-Rest bezeichnet, wobei der Alkylrest wie vorstehend definiert ist;
ein Arylrest einen aromatischen Rest mit fünf bis fünfzehn Kohlenstoffatomen darstellt, welcher mit einem oder mehreren Substituenten R³ substituiert sein kann, wobei R³ wie vorstehend definiert ist;
ein Heteroarylrest einen 5- oder 6-gliedrigen heterocyclischen Rest darstellt, welcher mindestens ein aus O, N und S ausgewähltes Heteroatom enthält, wobei der heterocyclische Rest an einen weiteren Ring anelliert sein kann;
mit der Maßgabe, dass die Behandlung des menschlichen oder tierischen Körpers ausgeschlossen ist.

11. Verwendung gemäß Anspruch 10, wobei das Medikament, das antibakterielle Mittel oder die Antifouling-Beschichtung zur Behandlung oder Vorbeugung von durch Gramnegativen Bakterien verursachten bakteriellen Schäden und Erkrankungen ist.

12. Verwendung gemäß Anspruch 10, wobei das Medikament, das antibakterielle Mittel oder die Antifouling-Beschichtung zur Behandlung oder Vorbeugung von durch *Pseudomonas aeruginosa* oder *Burkholderia cepacia* verursachten bakteriellen Schäden und Erkrankungen ist.

13. Verbindung der allgemeinen Formel (X) und pharmazeutisch verträglichen Salzen davon: 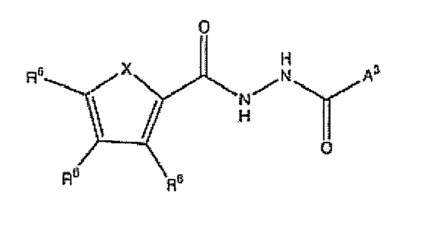 wobei
A³ ein C₈-C₂₀-Alkyl-, C₈-C₂₀-Alkenyl- oder C₈-C₂₀-Alkinyl-Rest oder 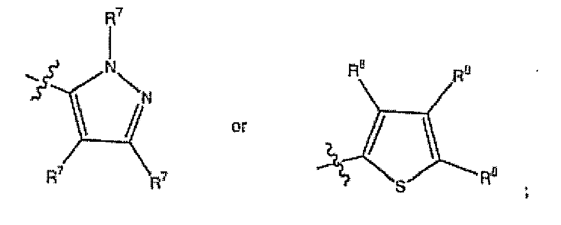 ist;
R⁶ unabhängig voneinander -H, -F, -Cl, -Br, -I, -NO₂, NR⁴R⁵, -CN, Alkyl, Cylcoalkyl, -OH, Alkoxy, Alkylthio, Hydroxyalkylamino, Haloalkyl, Haloalkyloxy, Hydroxyalkyl, Aryl, Alkenyl, Alkinyl oder Heteroaryl ist;
R⁷ unabhängig voneinander -H, -F, -Cl, -Br, -I, -NO₂, -NR⁴R⁵, -CN, Alkyl, Cylcoalkyl, -OH, Alkoxy, Alkylthio, Hydroxyalkylamino, Haloalkyl, Haloalkyloxy, Hydroxyalkyl, Aryl, Alkenyl, Alkinyl oder Heteroaryl ist;
R⁸ unabhängig voneinander -H, -F, -Cl, -Br, -I, -NO₂, -NR⁴R⁵, -CN, Alkyl, Cylcoalkyl, -OH, Alkoxy, Alkylthio, Hydroxyalkylamino, Haloalkyl, Haloalkyloxy, Hydroxyalkyl, Aryl, Alkenyl, Alkinyl oder Heteroaryl ist;
X ausgewählt ist aus S, O, N, NR⁴, SO oder SO₂;
R⁴ Wasserstoff, Alkyl, Alkenyl, Alkinyl, Cycloalkyl, Aryl oder Heteroaryl ist;
R⁵ Wasserstoff, O-Alkyl, O-Aryl, Alkyl, Alkenyl, Alkinyl, Heteroaryl oder Aryl ist;
wobei
ein Alkylrest einen linearen oder verzweigten C₁-C₆-Alkylrest bezeichnet, welcher mit einem oder mehreren Substituenten R³ substituiert sein kann;
ein Alkenylrest einen linearen oder verzweigten C₁-C₆-Alkenylrest bezeichnet, welcher mit einem oder mehreren Substituenten R³ substituiert sein kann;
ein Alkinylrest einen linearen oder verzweigten C₁-C₆-Alkinylrest bezeichnet, welcher mit einem oder mehreren Substituenten R³ substituiert sein kann;
ein Cycloalkylrest ein drei bis acht Kohlenstoffatome enthaltendes nichtaromatisches Ringsystem bezeichnet, wobei eines oder mehrere der Kohlenstoffatome im Ring durch eine Gruppe X substituiert sein können, wobei X wie vorstehend definiert ist;
ein Alkoxyrest einen O-Alkylrest darstellt, wobei der Alkylrest wie vorstehend definiert ist;
ein Haloalkylrest einen mit einem bis fünf Halogenatomen substituierten Alkylrest bezeichnet, wobei der Alkylrest wie vorstehend definiert ist;
ein Hydroxyalkylrest einen HO-Alkylrest bezeichnet, wobei der Alkylrest wie vorstehend definiert ist;
ein Haloalkyloxyrest einen mit einem bis fünf Halogenatomen substituierten Alkoxyrest bezeichnet, wobei der Alkoxyrest wie vorstehend definiert ist;
ein Hydroxyalkylaminorest einen (HO-Alkyl)₂N-Rest oder HO-Alkyl-NH-Rest bezeichnet, wobei der Alkylrest wie vorstehend definiert ist;
ein Arylrest einen aromastischen Rest mit fünf bis fünfzehn Kohlenstoffatomen darstellt, welcher mit einem oder mehreren Substituenten R³ substituiert sein kann, wobei R³ wie vorstehend definiert ist;
ein Heteroarylrest einen 5- oder 6-gliedrigen heterocyclischen Rest darstellt, welcher mindestens ein aus O, N und S ausgewähltes Heteroatom enthält, wobei der heterocyclische Rest an einen weiteren Ring anelliert sein kann;
mit der Maßgabe, dass 2-Thiophencarbonsäure-5-nitro-2-(2-thienylcarbonyl)hydrazid, 4-Butylthiophen-2-carbonsäure-N'-(4-butyl-thiophen-2-carbonyl)hydrazid, 2-Thiophencarbonsäure-3-chlor-2-(2-thienylcarbonyl)hydrazid, 2-Thiophencarbonsäure-4-brom-2-(2-thienylcarbonyl)hydrazid, 1 H-Pyrazol-5-carbonsäure-1-methyl-2-(2-thienyl-carbonyl)hydrazid, 2-Thiophendicarbonsäure-5(4,5,6,7-tetrahydrobenzo[b]thien-2-yl)-2-[[5-(4,5,6,7-tetrahydrobenzo[b]thien-2-yl)-2-thienyl]carbonyl]hydrazid, 1H-Pyrrol-2-carbonsäure-2-(2-thienylcarbonyl)hydrazid, 2-Thiaphencarbonsäure-2-(2-thienylcarbonyl)hydrazid, 2-Thiophencarbonsäure-N'-(furan-2-carbonyl)hydrazid, Thiophen-2-carbonsäure-N'-(3-chlor-4-methylthiophen-2-carbonyl)hydrazid, 2-Furancarbonsäure-5-[[[4-rnethyl-4-(trifluormethyl)-2-pyrimidinyl]thio]methyl]-2-(2-thienyl-carbonyl)hydrazid, 1H-Pyrazol-3-carbonsäure-4-brom-1,5-dimethyl-2-(2-thienylcarbonyl)hydrazid und 2-Thiophencarbonsäure-N'-(5-bromfuran-2-carbonyl)hydrazid ausgeschlossen sind.

14. Verbindung gemäß Anspruch 13, einschließlich der durch Disclaimer ausgeschlossenen Verbindungen, zur Verwendung als Medikament, antibakterielles Mittel oder Antifouling-Mittel.

15. Verwendung einer Verbindung gemäß Anspruch 13, einschließlich der durch Disclaimer ausgeschlossenen Verbindungen, zur Herstellung eines Medikaments, antibakteriellen Mittels oder einer Antifouling-Beschichtung zur Behandlung oder Vorbeugung von bakteriellen Schäden und Erkrankungen.

16. Verwendung gemäß Anspruch 15, wobei das Medikament, das antibakterielle Mittel oder die Antifouling-Beschichtung zur Behandlung oder Vorbeugung von durch Gram-negative Bakterien verursachten bakteriellen Schäden und Erkrankungen ist.

17. Verwendung gemäß Anspruch 15, wobei das Medikament, das antibakterielle Mittel oder die Antifouling-Beschichiung zur Behandlung oder Vorbeugung von durch *Pseudomonas aeruginosa* oder *Burkholderia cepacia* verursachten bakteriellen Schäden und Erkrankungen ist.

18. Verwendung einer Verbindung gemäß Anspruch 13 zur Behandlung von Biofilmen oder zur Inhibierung von Biofilm-Bildung.

19. Verwendung gemäß Anspruch 18 zur Behandlung von Biofilmen oder zur Inhibierung von Biofilm-Bildung auf medizinischen Gegenständen, Instrumenten und Geräten.

20. Verwendung gemäß Anspruch 18 zur Behandlung von Biofilmen oder zur Inhibierung von Biofilm-Bildung in Desinfektionsmitteln, Reinigungs- und Behazzdlungslösungen.

21. Verwendung gemäß Anspruch 18 zur Behandlung von Biofilmen oder zur Inhibierung von Biofilm-Bildung bei Gegenständen für die Körperpflege, Toilettenartikeln und Kosmetika.

22. Verwendung gemäß Anspruch 18 zur Behandlung von Biofilmen oder zur Inhibierung von Biofilm-Bildung im industriellen Bereich.

23. Verwendung gemäß Anspruch 18 zur Behandlung von Biofilmen oder zur inhibierung von Biofilm-Bildung im industriellen Bereich, wobei der industrielle Bereich ausgewählt ist aus Schiffskörpern, Nahrungsmittel-Verarbeitungssystemen, Ölgewinnungs- oder Papierherstellungsanlagen.

24. Verwendung gemäß Anspruch 18 zur Behandlung von Biofilmen oder zur Inhibierung von Biofilm-Bildung im Umweltbereich.

25. Verwendung gemäß Anspruch 18 zur Behandlung von Biofilmen oder zur Inhibierung von Biofilm-Bildung im Umweltbereich, wobei der Umweltbereich ausgewählt ist aus Wasserverteilungs- oder Kühlwassersystemen.

## Revendications

1. Utilisation des composés de formule générale (I) 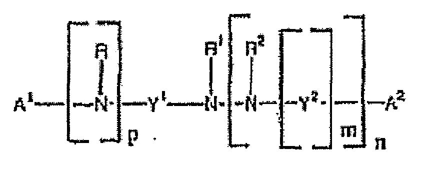 pour la régulation du système de détection du quorum de micro-organismes, dans laquelle, dans la formule (I),
R¹ est H ;
R2 est H ;
A¹ et A² représentent chacun indépendamment un groupe alkyle en C₁ à C₂₀, alcényle en C₁ à C₂₀ ou alcynyle en C₁ à C₂₀, qui peut contenir un ou plusieurs groupe(s) Z, ou un système cyclique aromatique ou non aromatique monocyclique ou polycyclique qui peut contenir un ou plusieurs groupe(s) X, et dans le cas d'un système cyclique polycyclique, ledit système contenant au moins un cycle aromatique ;
Z est choisi dans le groupe comprenant S, O, N, NR⁴, CO, CO₂, CS, SO ou SO₂ ;
X est choisi dans le groupe comprenant S, O, N, NR⁴, SO ou SO₂ ;
ledit système cyclique pouvant porter un substituant R³ sur un ou plusieurs des atomes de carbone dudit système cyclique ;
ledit groupe alkyle en C₁ à C₂₀, alcényle en C₁ à C₂₀ ou alcynyle en C₁ à C₂₀ pouvant porter un substituant R³ sur un ou plusieurs des atomes de carbone dudit groupe ;
R³ est indépendamment H, OR⁴, SR⁴, un groupe hydroxyalkyle, hydroxyalkylamine, cycloalkyle, un atome d'halogène, un groupe halogénoalkyle, halogénoalkyloxy, NO₂ CN, SO₂NR⁴R⁵, CO₂NR⁴R⁵, COR⁴, CO₂R⁴, SO₂R⁴, SO₃R⁴, NR⁴R⁵, un groupe alkyle, alcényle, alcynyle, aryle ou hétéroaryle ;
R⁴ est H, un groupe alkyle, alcényle, acynyle, cycloalkyle, aryle ou hétéroaryle ;
R⁵ est H, un groupe O-alkyle, O-aryle, alkyle, alcényle, alcynyle, hétéroaryle ou aryle ;
Y¹ et Y² sont C=O ;
p vaut 0, m vaut 1, n vaut 1 ;
dans laquelle
un groupe alkyle désigne un groupe alkyle en C₁ à C₆ linéaire ou ramifié qui peut être substitué par un ou plusieurs substituants R³ ;
un groupe alcényle désigne un groupe alcényle en C₁ à C₆ linéaire ou ramifié, qui peut être substitué par un ou plusieurs substituants R³ ;
un groupe alcynyle désigne un groupe alcynyle en C₁ à C₆ linéaire ou ramifié, qui peut être substitué par un ou plusieurs substituants R³ ;
un groupe cycloalkyle désigne un système cyclique non aromatique contenant trois à huit atomes de carbone, un ou plusieurs des atomes de carbone dans le cycle pouvant être substitué(s) par un groupe X, X étant tel que défini ci-dessus ;
un groupe alcoxy désigne un groupe O-alkyle, le groupe alkyle étant tel que défini ci-dessus ;
un groupe halogénoalkyle désigne un groupe alkyle qui est substitué par un à cinq atomes d'halogène, le groupe alkyle étant tel que défini ci-dessus ;
un groupe hydroxyalkyle désigne un groupe HO-alkyle, le groupe alkyle étant tel que défini ci-dessus ;
un groupe halogénoalkyloxy désigne un groupe alcoxy qui est substitué par un à cinq atomes de carbone, le groupe alkyle étant tel que défini ci-dessus ;
un groupe hydroxyalkylamino désigne un groupe (HO-alkyl)₂-N ou un groupe HO-alkyl-NH, le groupe alkyle étant tel que défini ci-dessus ;
un groupe aryle désigne un groupe aromatique ayant cinq à quinze atomes de carbone qui peut être substitué par un ou plusieurs substituants R³, R³ étant tel que défini ci-dessus ;
un groupe hétéroaryle désigne un groupe hétérocyclique de 5 ou 6 chaînons qui contient au moins un hétéroatome choisi parmi O, N et S, le groupe hétérocyclique pouvant être fusionné à un autre cycle ;
à condition que le traitement du corps humain ou animal soit exclu.

2. Utilisation selon la revendication 1, pour le traitement de biofilms ou pour l'inhibition de la formation de biofilms.

3. Utilisation selon la revendication 1 ou 2, pour le traitement de biofilms ou pour l'inhibition de la formation de biofilms sur des articles, instruments et dispositifs médicaux.

4. Utilisation selon la revendication 1 ou 2, pour le traitement de biofilms ou pour l'inhibition de la formation de biofilms dans des solutions désinfectantes, nettoyantes et de traitement.

5. Utilisation selon la revendication 1 ou 2, pour le traitement de biofilms ou pour l'inhibition de la formation de biofilms dans des articles d'hygiène personnelles, des produits de toilettes et des cosmétiques.

6. Utilisation selon la revendication 1 ou 2, pour le traitement de biofilms ou pour l'inhibition de la formation de biofilms dans les installations industrielles.

7. Utilisation selon la revendication 1 ou 2, pour le traitement de biofilms ou pour l'inhibition de la formation de biofilms dans les installations industrielles, l'installation industrielle étant choisie dans le groupe comprenant les coques de navires, les systèmes de traitement alimentaire, les installations d'extraction d'huile ou de fabrication du papier.

8. Utilisation selon la revendication 1 ou 2, pour le traitement de biofilms ou pour l'inhibition de la formation de biofilms dans des installations environnementales.

9. Utilisation selon la revendication 1 ou 2, pour le traitement de biofilms ou pour l'inhibition de la formation de biofilms dans des installations environnementales, les installations environnementales étant choisies dans le groupe comprenant les systèmes de distribution d'eau ou les systèmes de refroidissement par eau.

10. Utilisation des composés de formule générale (I) 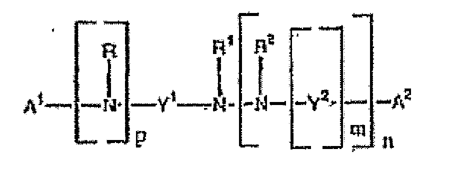 pour la préparation d'un médicament, d'un agent anti-bactérien ou d'un revêtement anti-salissure pour le traitement ou la prévention des dommages bactériens et des maladies,
dans laquelle, dans la formule (I)
R¹ est H ;
R² est H ;
A¹ et A² représentent chacun indépendamment un groupe alkyle en C₁ à C₂₀, alcényle en C₁ à C₂₀ ou alcynyle en C₁ à C₂₀, qui peut contenir un ou plusieurs groupe(s) Z, ou un système cyclique aromatique ou non aromatique monocyclique ou polycyclique qui peut contenir un ou plusieurs groupe(s) X, et dans le cas d'un système cyclique polycyclique, ledit système contient au moins un cycle aromatique;
Z est choisi dans le groupe comprenant S, O, N, NR⁴, CO, CO₂, CS, SO ou SO₂ ;
X est choisi dans le groupe comprenant S, O, N, NR⁴, SO ou SO₂ ;
ledit système cyclique pouvant porter un substituant R³ sur un ou plusieurs des atomes de carbone dudit système cyclique ;
ledit groupe alkyle en C₁ à C₂₀, alcényle en C₁ à C₂₀ ou alcynyle en C₁ à C₂₀ pouvant porter un substituant R³ sur un ou plusieurs des atomes de carbone dudit groupe ;
R³ est indépendamment H, OR⁴, SR⁴, un groupe hydroxyalkyle, hydroxyalkylamine, cycloalkyle, un atome d'halogène, un groupe halagénoalkyle, halogénoalkyloxy, NO₂, CN, SO₂NR⁴R⁵, CO₂NR⁴R⁵, COR⁴, CO₂R⁴, SO₂R⁴, SO₃R⁴, NR⁴R⁵, un groupe alkyle, alcényle, alcynyle, aryle ou hétéroaryle ;
R⁴ est H, un groupe alkyle, alcényle, acynyle, cycloalkyle, aryle ou hétéroaryle ;
R⁵ est H, un groupe O-alkyle, O-aryle, alkyle, alcényle, alcynyle, hétéroaryle ou aryle ;
Y¹ et Y² sont C=O ;
p vaut 0, m vaut 1, n vaut 1 ;
dans laquelle
un groupe alkyle désigne un groupe alkyle en C₁ à C₆ linéaire ou ramifié qui peut être substitué par un ou plusieurs substituants R³ ;
un groupe alcényle désigne un groupe alcényle en C₁ à C₆ linéaire ou ramifié, qui peut être substitué par un ou plusieurs substituants R³ ;
un groupe alcynyle désigne un groupe alcynyle en C₁ à C₆ linéaire ou ramifié, qui peut être substitué par un ou plusieurs substituants R³ ;
un groupe cycloalkyle désigne un système cyclique non aromatique contenant trois à huit atomes de carbone, un ou plusieurs des atomes de carbone dans le cycle pouvant être substitué(s) par un groupe X, X étant tel que défini ci-dessus ;
un groupe alcoxy désigne un groupe O-alkyle, le groupe alkyle étant tel que défini ci-dessus ;
un groupe halogénoalkyle désigne un groupe alkyle qui est substitué par un à cinq atomes d'halogène, le groupe alkyle étant tel que défini ci-dessus ;
un groupe hydroxyalkyle désigne un groupe HO-alkyle, le groupe alkyle étant tel que défini ci-dessus ;
un groupe halogénoalkyloxy désigne un groupe alcoxy qui est substitué par un à cinq atomes de carbone, le groupe alkyle étant tel que défini ci-dessus ;
un groupe hydroxyalkylamino désigne un groupe (HO-alkyl)₂-N ou un groupe HO-alkyl-NH, le groupe alkyle étant tel que défini ci-dessus ;
un groupe aryle désigne un groupe aromatique ayant cinq à quinze atomes de carbone qui peut être substitué par un ou plusieurs substituants R³, R³ étant tel que défini ci-dessus ;
un groupe hétéroaryle désigne un groupe hétérocyclique de 5 ou 6 chaînons qui contient au moins un hétéroatome choisi parmi O, N et S, le groupe hétérocyclique pouvant être fusionné à un autre cycle ;
à condition que le traitement du corps humain ou animal soit exclu.

11. Utilisation selon la revendication 10, dans laquelle le médicament, l'agent anti-bactérien ou le revêtement anti-salissure est destiné au traitement ou à la prévention des dommages bactériens et des maladies provoquées par des bactéries à Gram négatif.

12. Utilisation selon la revendication 10, dans laquelle le médicament, l'agent anti-bactérien ou le revêtement anti-salissure est destiné au traitement ou à la prévention des dommages bactériens et des maladies provoquées par *Pseudomonas aeruginosa* ou *Burkholderia cepacia.*

13. Composé de formule générale (X) et ses sels pharmaceutiquement acceptables : 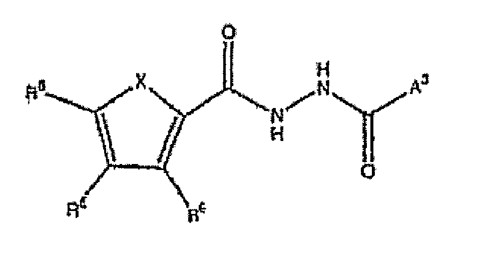 dans laquelle
A³ est un groupe alkyle en C₈ à C₂₀, alcényle en C₈ à C₂₀ ou alcynyle en C₈ à C₂₀ ou 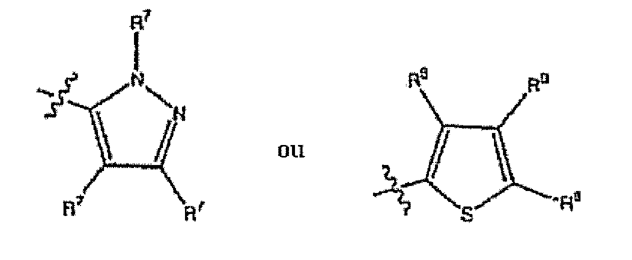 les R⁶ sont indépendamment les uns des autres, -H, -F, -Cl, -Br, -I, -NO₂, -NR⁴R⁵, -CN, un groupe alkyle, cycloalkyle, -OH, alcoxy, alkylthio, hydroxyalkylamino, halogénoalkyle, halogénoalkyloxy, hydroxyalkyle, aryle, alcényle, alcynyle ou hétéroaryle ;
les R⁷ sont indépendamment les uns des autres, -H, -F, -Cl, -Br, -I, -NO₂, -NR⁴R⁵, -CN, un groupe alkyle, alcényle, alcynyle, cycloalkyle, -OH, alcoxy, alkylthio, hydroxyalkylamino, halogénoalkyle, halogénoalkyloxy, hydroxyalkyle, aryle ou hétéroaryle ;
les R⁸ sont indépendamment les uns des autres, -H, -F, -Cl, -Br, -I, -NO₂, -NR⁴R⁵, -CN, un groupe alkyle, alcényle, alcynyle, cycloalkyle, -OH, alcoxy, alkylthio, hydroxyalkylamino, halogénoalkyle, halogénoalkyloxy, hydroxyalkyle, aryle ou hétéroaryle ;
X est choisi dans le groupe comprenant S, O, N, NR⁴, SO ou SO₂;
R⁴ est H, un groupe alkyle, alcényle, alcynyle, cycloalkyle, aryle ou hétéroaryle ;
R⁵ est H, un groupe O-alkyle, O-aryle, alkyle, alcényle, alcynyle, hétéroaryle ou aryle ; dans laquelle
un groupe alkyle désigne un groupe alkyle en C₁ à C₆ linéaire ou ramifié qui peut être substitué par un ou plusieurs substituants R³;
un groupe alcényle désigne un groupe alcényle en C₁ à C₆ linéaire ou ramifié, qui peut être substitué par un ou plusieurs substituants R³ ;
un groupe alcynyle désigne un groupe alcynyle en C₁ à C₆ linéaire ou ramifié, qui peut être substitué par un ou plusieurs substituants R³ ;
un groupe cycloalkyle désigne un système cyclique non aromatique contenant trois à huit atomes de carbone, un ou plusieurs des atomes de carbone dans le cycle pouvant être substitué(s) par un groupe X, X étant tel que défini ci-dessus ;
un groupe alcoxy désigne un groupe O-alkyle, le groupe alkyle étant tel que défini ci-dessus ;
un groupe halogénoalkyle désigne un groupe alkyle qui est substitué par un à cinq atomes d'halogène, le groupe alkyle étant tel que défini ci-dessus ;
un groupe hydroxyalkyle désigne un groupe HO-alkyle, le groupe alkyle étant tel que défini ci-dessus ;
un groupe halogénoalkyloxy désigne un groupe alcoxy qui est substitué par un à cinq atomes de carbone, le groupe alkyle étant tel que défini ci-dessus ;
un groupe hydroxyalkylamino désigne un groupe (HO-alkyl)₂-N ou un groupe HO-alkyl-NH, le groupe alkyle étant tel que défini ci-dessus ;
un groupe aryle désigne un groupe aromatique ayant cinq à quinze atomes de carbone qui peut être substitué par un ou plusieurs substituants R³, R³ étant tel que défini ci-dessus ;
un groupe hétéroaryle désigne un groupe hétérocyclique de 5 ou 6 chaînons qui contient au moins un hétéroatome choisi parmi O, N et S, le groupe hétérocyclique pouvant être fusionné à un autre cycle ;
à condition que l'acide 2-thiophène-carboxylique-5-nitro-2-(2-thiénylcarbonyl)hydrazide, l'acide 4-butylthiophène-2-carboxylique-N'-(4-butyl-thiophène-2-carbonyl)hydrazide, l'acide 2-thiophènecarboxylique-3-chloro-2-(2-thiénylcarbonyl)hydrazide, l'acide 2-thiophène-carboxylique-5-bromo-2-(2-thiénylcarbonyl)hydrazide, l'acide 1H-pyrazole-5-carboxylique-1-méthyl-2-(2-thiénylcarbonyl)hydrazide, l'acide 2-thiophènedicarboxylique-5-(4,5,6,7-tétrahydro-benzo[b]thién-2-yl)-2-[[5-(4,5,6,7-tétrahydrobenzo[b]thién-2-yl)-2-thiényl]-carboxyl]-hydroazide, l'acide 1H-pyrole-2-carboxylique-2-(2-thiénylcarbonyl)-hydrazide, l'acide 2-thiophène-carboxylique-2-(2-thiénylcarbonyl)-hydrazide, l'acide 2-thiophène-carboxylique-N'-(furane-2-carbonyl)hydrazide, l'acide thiophène-2-carboxylique-N'-(3-chloro-4-méthylthiophène-2-carbonyl)hydrazide, l'acide 2-furane-carboxylique-5-[[4-méthyl-6-(trifluorométhyl)-2-pyrimidinyl]thio]méthyl]-2-(2-thiénylcarbonyl)hydrazide, l'acide 1H-pyrazole-3-carboxylique-4-bromo-1,5-diméthyl-2-(2-thiénylcarbanyl)hydrazide et l'acide 2-thiaphène-carboxylique-N'-(5-bromofurane-2-carbanyl)hydrazide soient exclus.

14. Composé selon la revendication 13, comprenant les composés exclus par l'un quelconque des disclaimers, pour l'utilisation en tant que médicament, agent anti-bactérien ou agent anti-salissure.

15. Utilisation d'un composé selon la revendication 13, comprenant les composés exclus par l'un quelconque des disclaimers, pour la préparation d'un médicament, d'un agent anti-bactérien ou d'un revêtement anti-salissure, pour le traitement ou la prévention de dommages bactériens ou de maladies.

16. Utilisation selon la revendication 15, dans laquelle le médicament, l'agent antibactériens ou le revêtement anti-salissure est destiné au traitement ou à la prévention des dommages bactériens et des maladies provoquées par des bactéries à Gram négatif.

17. Utilisation selon la revendication 15, dans laquelle le médicament, l'agent anti-bactérien ou le revêtement anti-salissure est destiné au traitement ou à la prévention des dommages bactériens et des maladies provoquées par *Pseudomonas aeruginosa* ou *Burkholderia cepacia.*

18. Utilisation d'un composé selon la revendication 13, pour le traitement de biofilms ou pour l'inhibition de la formation de biofilms.

19. Utilisation selon la revendication 18, pour le traitement de biofilm ou pour l'inhibition de la formation de biofilms sur des articles, instruments et dispositifs médicaux.

20. Utilisation selon la revendication 18, pour le traitement de biofilms ou pour l'inhibition de la formation de biofilms dans des solutions désinfectantes, nettoyantes et de traitement.

21. Utilisation selon la revendication 18, pour le traitement de biofilms ou pour l'inhibition de la formation de biofilms dans des articles d'hygiène personnelles, de produits de toilette et de cosmétiques.

22. Utilisation selon la revendication 18, pour le traitement de biofilms ou pour l'inhibition de la formation de biofilms dans des installations industrielles.

23. Utilisation selon la revendication 18, pour le traitement de biofilms ou pour l'inhibition de la formation de biofilms dans les installations industrielles, l'installation industrielle étant choisie dans le groupe comprenant les coques de navires, les systèmes de traitement alimentaire, les installations d'extraction d'huile ou de fabrication du papier.

24. Utilisation selon la revendication 18, pour le traitement de biofilms ou pour l'inhibition de la formation de biofilms dans des installations environnementales.

25. Utilisation selon la revendication 18, pour le traitement de biofilms ou pour l'inhibition de la formation de biofilms dans des installations environnementales, les installations environnementales étant choisies dans le groupe comprenant les systèmes de distribution d'eau ou les systèmes de refroidissement par eau.
